(19) Europäisches Patentamt
European Patent Office
Office européen des brevets

(11) EP 4 248 949 B1

(12) EUROPEAN PATENT SPECIFICATION

(45) Date of publication and mention of the grant of the patent:
**28.08.2024 Bulletin 2024/35**

(21) Application number: **22163361.3**

(22) Date of filing: **21.03.2022**

(51) International Patent Classification (IPC):
***A61K 9/127*** *(2006.01)* ***A61K 31/517*** *(2006.01)*
***A61K 31/52*** *(2006.01)* ***A61K 31/675*** *(2006.01)*
***A61P 35/00*** *(2006.01)*

(52) Cooperative Patent Classification (CPC):
**A61K 9/1271; A61K 31/517; A61K 31/52; A61K 31/675; A61P 35/00**

# (54) LIPOSOMAL COMPOSITIONS

LIPOSOM-ZUSAMMENSETZUNGEN

COMPOSITIONS LIPOSOMALES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**27.09.2023 Bulletin 2023/39**

(73) Proprietor: **R.G.C.C. Holdings AG**
**6300 Zug (CH)**

(72) Inventor: **PAPASOTIRIOU, Ioannis**
**6315 Oberägeri (CH)**

(74) Representative: **Schmitz, Joseph**
**Isler & Pedrazzini AG**
**Giesshübelstrasse 45**
**Postfach 1772**
**8027 Zürich (CH)**

(56) References cited:
**WO-A1-2021/191236**

- **DAIKOPOULOU VASILIKI ET AL: "Targeting SARS-CoV-2 Polymerase with New Nucleoside Analogues", MOLECULES, vol. 26, no. 11, 7 June 2021 (2021-06-07), pages 3461, XP055955461, DOI: 10.3390/molecules26113461**
- **SALEHI BAHARE ET AL: "Liposomal Cytarabine as Cancer Therapy: From Chemistry to Medicine", BIOMOLECULES, vol. 9, no. 12, 23 November 2019 (2019-11-23), pages 773, XP055955508, DOI: 10.3390/biom9120773**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

TECHNICAL FIELD

The present invention relates to liposomal compositions, in particular liposomal compositions incorporating nucleoside analogues, and their use in cancer treatment.

PRIOR ART

Cancer, one of the leading causes of death worldwide, continues to be the focus of many research efforts.

While new potentially therapeutic molecules as well as new targets are discovered, the key to effectively treat a certain disorder, such as cancer, often depends on enhancing the delivery of an active ingredient to the targeted cells.

Innovative drug delivery systems that are capable of such delivery are needed and under rigorous research to provide biocompatible, cost effective, safe and efficient options that allow their use in therapy.

A prominent drug delivery system is that of liposomes, which can be loaded in their interior with active ingredients. Liposomes are nano-sized, and can be modified on their surface in order to be more targeted or multiple types of liposomes can be combined for improved results.

Liposomes are a well-established tool for drug delivery and were first described by Alee Bangham in 1963.

Today, there exist already products in liposomal form available commercially like Onivyde™, Marqibo®, Doxil®, Visudyne® and Depocyt®, which are useful in cancer therapy.

Liposomes encapsulate both hydrophilic and hydrophobic drugs. They are mainly composed of phospholipids, which can form single layers or bilayers in most environments. Hydrophilic drugs are usually entrapped in the interior of liposomes (aqueous environment) whereas hydrophobic molecules stay within the lipid bilayers of liposomes. When phospholipids disperse at an aqueous environment at temperatures above their transition temperature they tend to form particles with an aqueous core.

Liposomes have an additional advantage when used in combination with cytotoxic agents. The compartmentalization provided by the liposomes can reduce toxicity while at the same time offering a more targeted drug delivery and thus, increased efficacy.

WO2017155923A1 relates to nucleotide or nucleoside therapeutic compositions as well as their use in treating infectious diseases, viral infections, and cancer, and discloses that liposomal suspensions may be appropriate for the delivery of free nucleosides, acyl nucleosides or phosphate ester prodrug forms of the nucleoside compounds according to the present invention.

WO2015038596A1 relates to further to sulfur-containing nucleotide or nucleoside therapeutic compositions as well as their use in treating infectious diseases, viral infections, and cancer, and discloses that liposomal suspensions may be appropriate for the delivery of free nucleosides, acyl nucleosides or phosphate ester prodrug forms of the nucleoside compounds according to the present invention.

WO2010014895A2 relates to nucleic acids, such as oligonucleotides, that are encapsulated within liposomes complexes containing a mixture of a cationic lipid, a fusogenic lipid and a PEG lipid.

WO2006029081A2 relates to nucleoside-lipid conjugates comprising nucleosides and nucleoside analogues such as gemcitabine conjugated with phospholipids and further relates to the use of such nucleoside-lipid conjugates for the treatment of mammalian diseases, such as cancer and viral infections. The nucleoside-lipid conjugates may be used in the preparation of liposomes together with phospholipids such as phosphatidylcholine, phosphatidylethanolamine, phosphatidylglycerol, phosphatidic acid, cholesterol, [alpha]-tocopherol, natural or synthetic cardiolipin and analogues thereof, synthetic cationic cardiolipin analogues and derivatives thereof, phosphatidylserine, phosphatidylinositol, sphingomyelin, lysophosphotidylglycerol, lysophosphatidic acid, lysophosphotidylcholine, lysophosphatidylserine and PEG modified lipids.

WO2011062503A1 relates to parenteral formulations for certain long chain saturated and monounsaturated fatty acid derivatives of 2',2'-difluorodeoxycytidine (Gemcitabine), a nucleoside analog, in the treatment of cancer, and further relates to a parenteral pharmaceutical composition said derivatives in the form of liposomes having an average particle size in the range of 2.5-30 nm.

US20030026831 A1 relates to a pharmaceutical composition comprising: (a) an anionic liposome comprising a phospholipid with a head group selected from the group consisting of sn-glycero-phosphocholine; sn-glycero-phospho-rac-(1-glycer- ol); sn-glycero-phospho-L-serine; sn-glycero-3-phosphate; and combinations thereof; (b) a bioactive agent, which may be a nucleoside analogue; and (c) a cation, a buffer, or a combination thereof; wherein the anionic liposome is not a combination of 1,2-dioleoyl-sn-glycero-3-phosphocholine (DOPC) and 1,2-dioleoyl-sn-glycero-3-[phospho-L-serine] (DOPS) in a ratio of 2:1 having a diameter of 122 nm to 162 nm.

Saraf, Shivani, et al. "Advances in liposomal drug delivery to cancer: An overview." *Journal of drug delivery science and technology* 56 (2020): 101549 is a review publication that discloses liposomes in cancer therapy, for example

for liposomes of paclitaxel, triptolide, resveratrol,doxorubicin, docetaxel, hydroxyquinone, curcumin, sorafenib, shikonin, losartan, and gemcitabine, 5-fluorouracil, cordycepin, . Gemcitabine is a pyrimidine analogue, in particular a nucleoside analogue, in which the hydrogen atoms on the 2' carbon of deoxycytidine are replaced by fluorine atoms. 5-fluorouracil is a pyrimidine analogue, in particular a nucleobase analogue. Cordycepin is a purine analogue, in particular a is a adenosine analogue.

SUMMARY OF THE INVENTION

It is therefore an object of the present invention to provide a pharmaceutical composition comprising a nucleoside analogue loaded into liposomes of a liposomal delivery system, wherein the nucleoside analogue is a purine analogue chosen from {[2-(6-amino-9H-purin-9-yl)quinazolin-4-yl]oxy}phosphonic acid or 2-(6-amino-9H-purin-9-yl) quinazolin-4-ol, or a pyrimidine analogue chosen from 4-amino-1-(4-hydroxyquinazolin-2-yl)-1,2-dihydropyrimidin-2-one or N-[1-(4-hydroxyquinazolin-2-yl)-2-oxo-1,2-dihydropyrimidin-4-yl]acetamide. The aforementioned nucleoside analogues were hitherto unknown for use in a pharmaceutical composition, in particular for use in the treatment of cancer.

The chemical structure of the nucleoside analogues are depicted below.

| Name | Chemical structure |
|---|---|
| {[2-(6-amino-9H-purin-9-yl)quinazolin-4-yl]oxy}phosphonic acid | 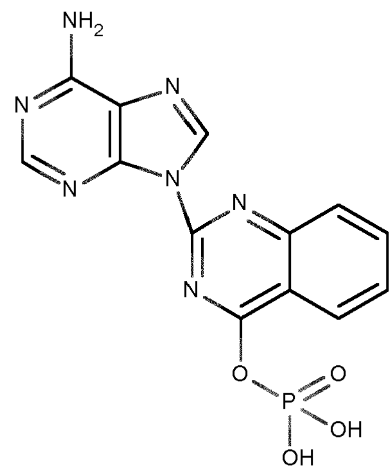 |
| 4-amino-1-(4-hydroxyquinazolin-2-yl)-1,2-dihydropyrimidin-2-one | 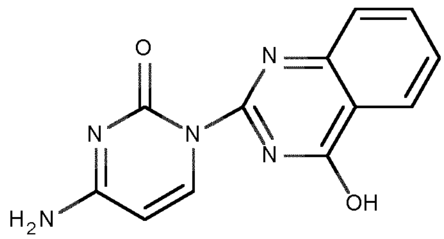 |
| 2-(6-am ino-9H-purin-9-yl)quinazolin-4-ol | 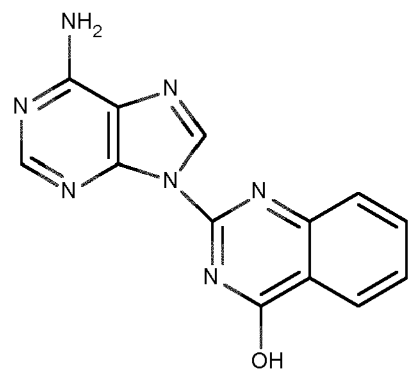 |

(continued)

| Name | Chemical structure |
|---|---|
| N-[1-(4-hydroxyquinazolin-2-yl)-2-oxo-1,2-dihydropyrimidin-4-yl] acetamide | 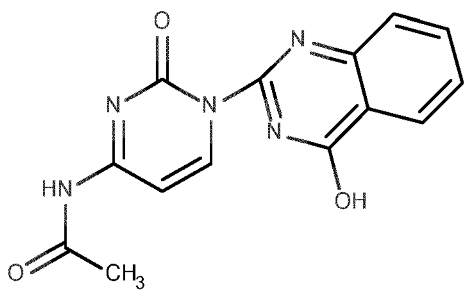 |

In an embodiment of the pharmaceutical composition according to the above object of the invention, the nucleoside analogue is covalently coupled or is not covalently coupled to the liposome.

In an embodiment of the pharmaceutical composition according to the above object of the invention, the liposomes of the liposomal delivery system have a diameter of at least 750, preferably of at least 775, and more preferably of at least 800 nm, and preferably of from 800 nm to 1000 nm, or 800 to 950 nm, as determined by SEM. Liposomes having the preferred size are more stable than liposomes having smaller sizes, especially than smaller liposomes having a size in the range 10 to 100 nm.

In an embodiment of the pharmaceutical composition according to the above object of the invention, the liposomes of the liposomal delivery system are sterically stabilized liposomes.

In an embodiment of the pharmaceutical composition according to the above object of the invention, the liposomes of the liposomal delivery system comprise at least a phospholipid, preferably at least a phosphatidylcholine and/or a phosphatidylethanolamine, or comprise at least a phospholipid, more preferably at least a phosphatidylcholine and/or a phosphatidylethanolamine, most preferably in addition to one or more phosphogylcerides and/or one or more sphingolipids. The inclusion of phosphatidylethanolamine and phosphatidylcholine has the advantage of providing amino or trimethylamino groups on the surfaces of the liposomes, which groups may be useful for further functionalizing the liposomes, for example via the formation of amide bonds in case of proteins or amino acids.

In an embodiment of the pharmaceutical composition according to the above object of the invention, the liposomes of the liposomal delivery system comprise, or essentially consist of, as the lipid component(s) of the liposomes, a pegylated phospholipid such as pegylated 1,2-distearoyl-sn-glycero-3-phosphoethanolamine, or a combination of a peglyated and an unpegylated phospholipid such as such as pegylated 1,2-distearoyl-sn-glycero-3-phosphoethanolamine and unpegylated phosphatidylcholine, respectively, optionally together with a sterol such as cholesterol. It has been found that liposomes of the aforementioned composition have better incorporation efficiency during the production process.

In an embodiment of the pharmaceutical composition according to the above object of the invention, the pegylated phospholipid is pegylated phosphoethanolamine and the unpegylated phospholipid is a phosphatidylcholine. It has been found that liposomes of the aforementioned composition have better incorporation efficiency during the production process.

In an embodiment of the pharmaceutical composition according to the above object of the invention, the liposomes of the liposomal delivery system are free of fatty acids in particular free of unsaturated, and preferably of monounsaturated, fatty acids such as oleic acid. It has been found that when liposomes are free of fatty acids, incorporation efficiency during the production process is enhanced.

In an embodiment of the pharmaceutical composition according to the above object of the invention, the nucleoside is {[2-(6-amino-9H-purin-9-yl)quinazolin-4-yl]oxy}phosphonic acid, and the liposomes of the liposomal delivery system comprise, or consists of, a pegylated phospholipid as the sole phospholipid component of the liposome, such as for example N-(carbonyl-methoxypolyethylene glycol-2000)-1,2-distearoyl-sn-glycero-3 phosphoethanolamine and a sterol, such as cholesterol. In that embodiment, in the liposome, the amount of pegylated phospholipid may of from about 85 wt% to about 95 wt%, more preferably of from about 90 wt% to about 95 wt%, based on the total weight of the pegylated phospholipid and the sterol. In that embodiment, in the liposome, the amount of sterol may of from about 5 wt% to about 15 wt%, more preferably of from about 5 wt% to about 10 wt%, based on the total weight of the pegylated phospholipid and the sterol.

In an embodiment of the pharmaceutical composition according to the above object of the invention, the liposomes of the liposomal delivery system comprise an amount of sterol such as cholesterol of from about more than 5 wt% to less than 50 wt%, preferably of from about more than 5 wt% to less than 25 wt%, more preferably of from about

more than 5 wt% to less than 20 wt%, most preferably of from about more than 6 wt% to less than 16 wt%, based on the weight of the pegylated phospholipid or the weight of the combination of a peglyated and an unpegylated phospholipid.

In an embodiment of the pharmaceutical composition according to the above object of the invention, the liposomes of the liposomal delivery system comprises, or consists of, of from 40 to 45 wt% of a pegylated phospholipid such as N-(carbonyl-methoxypolyethylene glycol-2000)-1,2-distearoyl-sn-glycero-3 phosphoethanolamine, of from 40 to 45 wt% of an unpegylated phospholipid such as phosphatidylcholine, and of a sterol such as cholesterol, based on the total weight of the pegylated phospholipid, unpegylated phospholipid and sterol, and preferably wherein the pegylated phospholipid and unpegylated phospholipid are comprised at a weight ratio of 1:1.

It is a further object of the present invention to provide a pharmaceutical composition according to the above object for use in the treatment of colon cancer or lung cancer or breast cancer, preferably via oral or parenteral route.

It is yet a further object of the present invention to provide aUse of a pharmaceutical composition according to the above object for the inhibition of proliferation of cancer cell lines *in vitro,* preferably in a concentration of 0.01μM to 10μM and/or wherein the cancer cell line is preferably a lung or colon or breast cancer cell line and/or the nucleoside analog is {[2-(6-amino-9H-purin-9-yl)quinazolin-4-yl]oxy}phosphonic acid or 4-amino-1-(4-hydroxyquinazolin-2-yl)-1,2-dihydropyrimidin-2-one, and is preferably present in a concentration of 0.01μM to 1μM.

It is another object of the present invention to provide a process for producing a liposomal delivery system comprising a nucleoside analogue loaded into liposomes, wherein the liposomes of the liposomal delivery system comprise, or consist of, a combination of a pegylated and an unpegylated phospholipid, and preferably of from 40 to 45 wt% of a pegylated phospholipid such as N-(carbonyl-methoxypolyethylene glycol-2000)-1,2-distearoyl-sn-glycero-3 phosphoethanolamine, of from 40 to 45 wt% of an unpegylated phospholipid such as phosphatidylcholine, and of a sterol such as cholesterol, based on the total weight of the pegylated phospholipid, unpegylated phospholipid and sterol, and more preferably wherein the pegylated phospholipid and unpegylated phospholipid are comprised at a weight ratio of 1:1, or 4:5 to 5:4,, and wherein the nucleoside analogue is chosen among 4-amino-1-(4-hydroxyquinazolin-2-yl)-1,2-dihydropyrimidin-2-one, or N-[1-(4-hydroxyquinazolin-2-yl)-2-oxo-1,2-dihydropyrimidin-4-yl]acetamide, wherein the process comprises the steps of:

a. solubilizing the pegylated phospholipid, unpegylated phospholipid, and sterol, and the nucleoside analogue, in an polar solvent, preferably in a mixture of polar solvents,
b. evaporating the solvent, and
c. adding an aqueous solution or water, preferably an aqueous buffer solution, such as to form a first liposomal suspension , and
d. sonicating the first liposomal suspension to form a second liposomal suspension,
e. annealing the second liposomal suspension to form the liposomal delivery system comprising a nucleoside analogue loaded into liposomes.

It is another object of the present invention to provide a process for producing a liposomal delivery system comprising a nucleoside analogue loaded into liposomes, wherein the liposomes of the liposomal delivery system comprise, or consist of, a combination of a pegylated and an unpegylated phospholipid, and preferably of from 40 to 45 wt% of a pegylated phospholipid such as N-(carbonyl-methoxypolyethylene glycol-2000)-1,2-distearoyl-sn-glycero-3 phosphoethanolamine, of from 40 to 45 wt% of an unpegylated phospholipid such as phosphatidylcholine, and of a sterol such as cholesterol, based on the total weight of the pegylated phospholipid, unpegylated phospholipid and sterol, and more preferably wherein the pegylated phospholipid and unpegylated phospholipid are comprised at a weight ratio of 1:1, or 4:5 to 5:4, and wherein the nucleoside analogue is chosen among {[2-(6-amino-9H-purin-9-yl)quinazolin-4-yl]oxy} phosphonic acid and 2-(6-Amino-9H-purin-9-yl) quinazolin-4-ol, wherein the process comprises the steps of:

a. solubilizing the pegylated phospholipid, unpegylated phospholipid, and sterol, in an polar solvent, preferably in a mixture of polar solvents,
b. evaporating the solvent, and
c. adding an aqueous solution of the nucleoside analogue , preferably an aqueous buffer solution of the nucleoside analogue, to form a first liposomal suspension, and
d. sonicating the first liposomal suspension to form a second liposomal suspension,
e. annealing the second liposomal suspension to form the liposomal delivery system comprising a nucleoside analogue loaded into liposomes.

It is another another object of the present invention to provide a process process for producing a liposomal delivery system comprising a nucleoside analogue loaded into liposomes,

wherein the liposomes of the liposomal delivery system comprise, or consist of, a pegylated phospholipid as the

sole phospholipid component of the liposome, such as N-(carbonyl-methoxypolyethylene glycol-2000)-1,2-distearoyl-sn-glycero-3 phosphoethanolamine, and of a sterol such as cholesterol, where in the liposome, preferably the amount of pegylated phospholipid is of from about 85 wt% to about 95 wt%, more preferably of from about 90 wt% to about 95 wt%, based on the total weight of the pegylated phospholipid and the sterol, and/or in the liposome, the amount of sterol is of from about 5 wt% to about 15 wt%, more preferably of from about 5 wt% to about 10 wt%, based on the total weight of the pegylated phospholipid and the sterol, and
wherein the nucleoside analogue is {[2-(6-amino-9H-purin-9-yl)quinazolin-4-yl]oxy}phosphonic acid,
wherein the process comprises the steps of:

a. solubilizing the pegylated phospholipid and sterol, in an polar solvent, preferably in a mixture of polar solvents,
b. evaporating the solvent, and
c. adding an aqueous solution of the nucleoside analogue, preferably an aqueous buffer solution of the nucleoside analogue, such as to form a first liposomal suspension, and
d. sonicating the first liposomal suspension to form a second liposomal suspension,
e. annealing the second liposomal suspension to form the liposomal delivery system comprising a nucleoside analogue loaded into liposomes.

Further embodiments of the invention are laid down in the dependent claims.

BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,

Fig. 1 shows a flow chart that depicts an embodiment of the claimed process, in which the encapsulation of 4-amino-1-(4-hydroxyquinazolin-2-yl)-1,2-dihydropyrimidin-2-one, and N-[1-(4-hydroxyquinazolin-2-yl)-2-oxo-1,2-dihydropyrimidin-4-yl]acetamide into the liposomal delivery is shown. Note that for 4-Amino-1-(4-hydroxyquinazolin-2-yl)-1,2-dihydropyrimidin-2-one, and N-[1-(4-hydroxyquinazolin-2-yl)-2-oxo-1,2-dihydropyrimidin-4-yl]acetamide, the nucleoside analogue is added at the stage of dissolution of the lipid components of the liposomal delivery system in chloroform:methanol.

Fig. 2 shows a flow chart that depicts an embodiment of the claimed process, in which the encapsulation of {[2-(6-amino-9H-purin-9-yl)quinazolin-4-yl]oxy}phosphonic acid and 2-(6-Amino-9H-purin-9-yl) quinazolin-4-ol into the liposomal delivery is shown. Note that for {[2-(6-amino-9H-purin-9-yl)quinazolin-4-yl]oxy}phosphonic acid and 2-(6-Amino-9H-purin-9-yl) quinazolin-4-ol, the nucleoside analogue is added at the stage of hydration of the thin film with PBS, the nucleoside analogue being dissolved in PBS.

Fig. 3 shows standard curves for of the different nucleoside analogues in phosphate buffer saline (PBS) for {[2-(6-amino-9H-purin-9-yl)quinazolin-4-yl]oxy}phosphonic acid, for 2-(6-Amino-9H-purin-9-yl) quinazolin-4-ol, for N-[1-(4-hydroxyquinazolin-2-yl)-2-oxo-1,2-dihydropyrimidin-4-yl]acetamide, for 4-amino-1-(4-hydroxyquinazolin-2-yl)-1,2-dihydropyrimidin-2-one

Fig.4 shows a differential scanning calorimetry thermographs of the phase transition peaks of liposomes of (1): 4-amino-1-(4-hydroxyquinazolin-2-yl)-1,2-dihydropyrimidin-2-one in liposomes of the liposomal delivery system 3, (2): blank liposomes of the liposomal delivery system 3, (3): 2-(6-Amino-9H-purin-9-yl) quinazolin-4-ol in liposomes of the liposomal delivery system 3, (4): blank liposomes of the liposomal delivery system 2, (5): N-[1-(4-hydroxyquinazolin-2-yl)-2-oxo-1,2-dihydropyrimidin-4-yl]acetamide in liposomes of the liposomal delivery system 3, (6):{[2-(6-amino-9H-purin-9-yl)quinazolin-4-yl]oxy}phosphonic acid in liposomes of the liposomal delivery system 2.

Fig.5 shows SEM images of blank liposomes, with 1a and 1b are pictures of liposome blank1 with diameters of 642, 630, and 668 nm as casn be seen in 1a, which is an enöarged portion of 1b and 2a and 2b are pictures of liposome blank 2 with diameters of 642, 630, and 668 nm.

Fig.6 shows SEM images of (A): liposomes encapsulating (A): 2-(6-Amino-9H-purin-9-yl) quinazolin-4-yl dihydrogen phosphate, with an enlarged section of the SEM image of (A) shown in (B). In (B), it can be seen that the diameter of three selected liposomes is determined to be 807, 816 and 838 nm.

Fig.7 shows SEM images of (A): liposomes encapsulating (A): 2-(6-Amino-9H-purin-9-yl) quinazolin-4-ol, with an enlarged section of the SEM image of (A) shown in (B). In (B), it can be seen that the diameter of three selected liposomes is determined to be 938, 907 and 925 nm.

Fig.8 shows SEM images of (A): liposomes encapsulating (A): and N-(9-(4-Hydroxyquinazolin-2-yl)-6-oxo-6,9-dihydro-1H-purin-2-yl)acetamide, with an enlarged section of the SEM image of (A) shown in (B). In (B), it can be

seen that the diameter of three selected liposomes is determined to be 860, 830 and 755 nm.

Fig.9 shows SEM images of (A): liposomes encapsulating 4-amino-1-(4-hydroxyquinazolin-2-yl)-1,2-dihydropyrimidin-2-one, with an enlarged section of the SEM image of (A) shown in (B). In (B), it can be seen that the diameter of three selected liposomes is determined to be 862, 846 and 834 nm.

DESCRIPTION OF PREFERRED EMBODIMENTS

In an embodiment of the pharmaceutical composition according to the above object of the invention, the nucleoside analogue is covalently coupled or is not covalently coupled to the liposome.

In an embodiment of the pharmaceutical composition according to the above object of the invention, the liposomes of the liposomal delivery system have a diameter of at least 750, preferably of at least 775, and more preferably of at least 800 nm, and preferably of from 800 nm to 1000 nm, or 800 to 950 nm, as determined by SEM. Liposomes having the preferred size are more stable than liposomes having smaller sizes, especially than smaller liposomes having a size in the range 10 to 100 nm.

In an embodiment of the pharmaceutical composition according to the above object of the invention, the liposomes of the liposomal delivery system are sterically stabilized liposomes.

In an embodiment of the pharmaceutical composition according to the above object of the invention, the liposomes of the liposomal delivery system comprise at least a phospholipid, preferably at least a phosphatidylcholine and/or a phosphatidylethanolamine, or comprise at least a phospholipid, more preferably at least a phosphatidylcholine and/or a phosphatidylethanolamine, most preferably in addition to one or more phosphogylcerides and/or one or more sphingolipids. The inclusion of phosphatidylethanolamine and phosphatidylcholine has the advantage of providing amino or trimethylamino groups on the surfaces of the liposomes, which groups may be useful for further functionalizing the liposomes, for example via the formation of amide bonds in case of proteins or amino acids.

In an embodiment of the pharmaceutical composition according to the above object of the invention, the liposomes of the liposomal delivery system comprise, or essentially consist of, as the lipid component(s) of the liposomes, a pegylated phospholipid such as pegylated 1,2-distearoyl-sn-glycero-3-phosphoethanolamine, or a combination of a peglyated and an unpegylated phospholipid such as pegylated 1,2-distearoyl-sn-glycero-3-phosphoethanolamine and unpegylated phosphatidylcholine, respectively, optionally together with a sterol such as cholesterol. It has been found that liposomes of the aforementioned composition have better incorporation efficiency during the production process.

In an embodiment of the pharmaceutical composition according to the above object of the invention, the pegylated phospholipid is pegylated phosphoethanolamine and the unpegylated phospholipid is a phosphatidylcholine. It has been found that liposomes of the aforementioned composition have better incorporation efficiency during the production process.

In an embodiment of the pharmaceutical composition according to the above object of the invention, the liposomes of the liposomal delivery system are free of fatty acids in particular free of unsaturated, and preferably of monounsaturated, fatty acids such as oleic acid. It has been found that when liposomes are free of fatty acids, incorporation efficiency during the production process is enhanced.

In an embodiment of the pharmaceutical composition according to the above object of the invention, the nucleoside is {[2-(6-amino-9H-purin-9-yl)quinazolin-4-yl]oxy}phosphonic acid, and the liposomes of the liposomal delivery system comprise, or consists of, a pegylated phospholipid as the sole phospholipid component of the liposome, such as for example N-(carbonyl-methoxypolyethylene glycol-2000)-1,2-distearoyl-sn-glycero-3 phosphoethanolamine and a sterol, such as cholesterol. In that embodiment, in the liposome, the amount of pegylated phospholipid may of from about 85 wt% to about 95 wt%, more preferably of from about 90 wt% to about 95 wt%, based on the total weight of the pegylated phospholipid and the sterol. In that embodiment, in the liposome, the amount of sterol may of from about 5 wt% to about 15 wt%, more preferably of from about 5 wt% to about 10 wt%, based on the total weight of the pegylated phospholipid and the sterol.

In an embodiment of the pharmaceutical composition according to the above object of the invention, the liposomes of the liposomal delivery system comprise an amount of sterol such as cholesterol of from about more than 5 wt% to less than 50 wt%, preferably of from about more than 5 wt% to less than 25 wt%, more preferably of from about more than 5 wt% to less than 20 wt%, most preferably of from about more than 6 wt% to less than 16 wt%, based on the weight of the pegylated phospholipid or the weight of the combination of a peglyated and an unpegylated phospholipid.

In an embodiment of the pharmaceutical composition according to the above object of the invention, the liposomes of the liposomal delivery system comprises, or consists of, of from 40 to 45 wt% of a pegylated phospholipid such as N-(carbonyl-methoxypolyethylene glycol-2000)-1,2-distearoyl-sn-glycero-3 phosphoethanolamine, of from 40 to 45 wt% of an unpegylated phospholipid such as phosphatidylcholine, and of a sterol such as cholesterol, based on the total weight of the pegylated phospholipid, unpegylated phospholipid and sterol, and preferably wherein the pegylated phospholipid and unpegylated phospholipid are comprised at a weight ratio of 1:1.

use in a pharmaceutical composition, in particular for use in the treatment of cancer.

It is understood that the above limitations to the composition apply also to the process for producing a liposomal delivery system comprising any of the nucleoside analogues.

In a preferred embodiment of the process for producing a liposomal delivery system comprising any of the nucleoside analogues loaded into liposomes, the polar solvent comprises chloroform, preferably mixed with ethanol or methanol, in a ratio of 3:1.

In a preferred embodiment of the process for producing a liposomal delivery system comprising any of the nucleoside analogues loaded into liposomes, annealing of the second liposomal suspension to form the liposomal delivery system comprising a nucleoside analogue loaded into liposomes is carried out at a temperature of at least 45°C, more preferably at a temperature of more than 50°C and most preferably at a temperature of between 55 and 75° such as for example at 65°C.

In a preferred embodiment of the process for producing a liposomal delivery system comprising any of the nucleoside analogues loaded into liposomes, the aqueous buffer solution is phosphate saline buffer, and preferably is a phosphate saline buffer having a neutral pH.

In a preferred embodiment of the process for producing a liposomal delivery system comprising any of the nucleoside analogues loaded into liposomes, the concentration of the nucleoside analogues in step c. is about 1 mg/mL in the first liposomal suspension.

EXAMPLES

## Materials and Methods

LIPOID PE 18:0/18:0 - PEG 2000, hereinafter DSPE-PEG, ([N-(carbonyl-methoxypolyethylene glycol-2000)-1,2-distearoyl-sn-glycero-3 phosphoethanolamine, Sodium Salt]) and LIPOID S PC-3, hereinafter PC, (Hydrogenated phosphatidylcholine from soybean) were purchased by Lipoid (Lipoid GmbH, Frigenstraße 4, D-67065 Ludwigshafen, Germany). Cholesterol, hereinafter CHOL, and Oleic acid, hereinafter OA, were purchased by Fluorochem (Graphite Way, Hadfield, Derbyshire, UK). Phosphate Buffer Saline 0.01M and pH 7.4 was purchased from Sigma-Aldrich (3050 Spruce Street, Saint Louis, MO 63103, USA) as well as Methanol and Chloroform.

## Preparation of Liposomes

Liposomes were prepared based on the thin film method. Different lipid concentrations were tested and based on their encapsulation efficacy, the ideal system was selected for each occasion. 2-(6-Amino-9H-purin-9-yl) quinazolin-4-ol and {[2-(6-amino-9H-purin-9-yl)quinazolin-4-yl]oxy}phosphonic acid were initially encapsulated into different systems and the ones that exhibited best results were used for the rest APIs. All the concentrations that were used, can be seen at Table 1.

**Table 1: Description of the different recipes that were used during the development of encapsulated nucleoside analogues into liposomes**

| **Code** | Composition | PC (100mg/mL), μL | DSPE-PEG (100mg/mL), μL | Cholesterol (20mg/mL), μL | Oleic Acid, μL |
|---|---|---|---|---|---|
| **1** | DSPE-PEG: CHOL:OA | - | 250 | 625 | 18.76 |
| **2** | DSPE-PEG: CHOL | - | 250 | 85 | - |
| | | | | | |
| **3** | DSPE-PEG:PC: CHOL | 125 | 125 | 196 | - |
| **4** | DSPE-PEG: CHOL:OA:PC | 125 | 125 | 625 | 18.76 |
| **5** | DSPE-PEG:OA | - | 250 | - | 18.76 |
| | | | | | |

Briefly, the preparation of liposomes began with the dissolution of PE 18:0/18:0-PEG 2000, Phosphatidylcholine (PC) and Cholesterol into 10 mL of a solution of chloroform: methanol (3:1) in a 50mL round bottom flask. Then, the solvent was removed through vacuum evaporation, until it was completely dried or in some cases for the complete evaporation of the solvent, nitrogen stream was used. After that, a thin film of lipid is formed at the walls of the flask, which is hydrated with 10mL Phosphate Buffer Saline (PBS) 0.01M pH 7.4, sonicated for 30 minutes at a sonication bath and then left to anneal its lipids at 65°C.

**Nucleoside Analogues encapsulation**

Nucleoside analogues were encapsulated based on their nature at different steps of the process. 4-amino-1-(4-hydroxyquinazolin-2-yl)-1,2-dihydropyrimidin-2-one, and N-[1-(4-hydroxyquinazolin-2-yl)-2-oxo-1,2-dihydropyrimidin-4-yl]acetamide were added initially at the process, along with the lipids and chloroform: methanol solution. To improve the solubility of the analogues, magnetic stirring was applied prior to vacuum evaporation (Process 1). On the other hand, {[2-(6-amino-9H-purin-9-yl)quinazolin-4-yl]oxy}phosphonic acid and 2-(6-Amino-9H-purin-9-yl) quinazolin-4-ol were added after their dissolution in PBS solution after the evaporation of the organic solvent (Process 2). The concentration of the nucleoside analogues was in all cases 1mg/mL. Figure 1 and 2 depict a schematic of liposome preparation according to Process 1 and 2, respectively.

As comparative examples, the same process as above was followed, but without adding any nucleoside analogues.

**Liposomes Purification**

At the end of the above encapsulation process, the solution was centrifuged at 15.000 rpm for 20 minutes and the supernatant was kept for indirect encapsulation determination. The precipitate was lyophilized and kept for further analysis and characterization.

**Freeze Drying**

Glass vials with rubber stoppers were inserted in the Freeze Dryer (Telstar, LyoBeta Mini) with liposomes precipitate in PBS. The freeze-drying process started with freezing for 2,5h at -40°C and hold of this temperature for other 3h. Then the condenser preparation starts and lasts for 10minutes. After that, the vacuum in the chamber begins, at 0,5mbar, in order to start drying. Primary drying lasts 2h and 13 min at 0°C and 0,5mbar. These conditions are hold for 11h (primary drying hold) and then secondary drying starts. Temperature during secondary drying is 23°C, 0,1mbar and lasts 6h and 17 min. Finally, cycle ends.

**Encapsulation Efficacy**

The supernatants after each centrifugation were kept and used to evaluate the amount of the free drug. Subtracting the amount of the free drug at the supernatant from the total added drug, the rest is considered as encapsulated. Briefly, from each solution samples were collected in order to prepare solutions, which were then evaluated with UV/VIS spectrometry. Based on standard curves, in which the different nucleosides were measured in solution on a UV-VIS spectrometer, different the remaining quantity was calculated. The encapsulation efficacy was measured then based on the following equation:

$$\frac{Quantity\ of\ the\ Nucleoside\ Analogue\ in\ the\ liposome}{Quantity\ of\ the\ initially\ added\ nucleoside\ analogue} x\ 100\ (1)$$

Quantity of the Nucleoside Analogue in the liposome = (Quantity of the Nucleoside Analogue initially added - Quantity of the Nucleoside Analogue in the supernatant).

**Yield**

After every completion of the freeze drying protocol, the dry powder of each product was weighed. The yield of each one was calculated based on the following equation (2):

$$\frac{Mass\ of\ the\ powder\ after\ freeze\ drying}{Mass\ of\ the\ intially\ added\ compounds}\ x\ 100\%\ (2)$$

### Scanning Electron Microscopy

Liposomes size, shape and appearance were evaluated using Scanning Electron Microscopy (SEM), Phenom ProX (Thermo Fischer Scientific). PELCO Tabs ™ Carbon Conductive Tabs, Double coated, 12mm OD were used with aluminum specimen mounts (1/2" slotted head and 1/8" pin). These tabs are adhesives that are used in SEM mounting for better conductivity. The chosen mode is mentioned as "all materials (5kV)", with an image intensity and BSD full detector. Live viewing has a resolution of 1024 and medium quality where the acquired image has 2048 resolution and high quality. The software version is 4.5.2. All image processing (photo acquisition, size analysis etc.) was done through the instrument's software.

### Differential Scanning Calorimetry

In general, thermal analysis measures the changes in properties of a material as a function of temperature. In this study, Differential Scanning Calorimetry was used, which measures the heat absorbed or liberated during heating or cooling. This happens through measuring the difference in heat flow rate between the sample and an inert reference under controlled atmosphere in correlation with time and temperature. DSC is a powerful tool when it comes to determining physical and chemical changes of materials and systems as a result of endothermic or exothermic events. The processes can be both exothermic or endothermic, depending whether the sampling is releasing or absorbing energy respectively. The basic principle of the method is that at physical transformations such as phase transition, more or less heat will need to flow to the sample than to the reference material to maintain both at the same temperature.

Differential Scanning Calorimetry (DSC) is a useful tool when it comes to liposome characterization. These colloidal lyotropic systems can show their properties through different temperature changes. The program used for this study starts with heating of the sample from 30°C to 180°C with a rate of 10°C/min and hold there for 1 minute. Then, heat is applied again from 180° to 370°C. The experiment is performed using DSC3, Mettler Toledo. The inert gas is $N_2$, with a rate of 50mL/min. Previously, the instrument was calibrated with a standard method using Indium as standard material. The software, with which the results are evaluated, is STARe Software.

### Cytotoxicity Assay

The human breast adenocarcinoma (luminal type) MCF7 cell line (ECACC 86012803), human breast adenocarcinoma (triple negative) MDA-MB-231 cell line (ECACC 92020424), human colorectal carcinoma HCT116 cell line (ECACC 91091005) and human lung cancer COLO699N cell line (ECACC 93052608) were culture in RPMI 1640 mediumCatalog#R0883 (Sigma-Aldrich, Darmstadt, Germany) supplemented with 10% fetal bovine serum Catalog#FB-1001/500 (BioSera, Nuaille, France) and 1% L-glutamine Catalog#G7513-100ML (Sigma-Aldrich, Darmstadt, Germany). All cell lines were maintained at 37°C in a humidified 5% $CO_2$ atmosphere. AlamarBlue assay was performed to assess cell viability. The viable cells were seeded at a density of 2 x $10^4$ (200 μl/well) in 96-well plate and incubated at 37°C and 5% CO2 for 24 h to form a cell monolayer. After 24 h of incubation, supernatant on the monolayer was aspirated and 200 μl of medium and varying concentrations of the substances were added and incubated for 24 h, 48 h and 72 h time points. After the specific time points, 20 μl of 0,15 mg/ml resazurin sodium salt Catalog#199303 (Sigma-Aldrich, Darmstadt, Germany) in PBS was added to each well and incubated for 3 h at 37°C and 5% $CO_2$. Fluorescence was measured at 560 nm excitation and 590nm emission wavelength. The experiment was performed in triplicates. The average fluorescence was calculated for each triplicate. One sample t-test was used to determine differences in the mean by comparing the treated samples with the untreated controls. P values < 0.05 were considered to indicate a statistically significant difference. Results were calculated using the Microsoft Excel 2016. The measurements from AlamarBlue assay were used to determine the LC50 (median lethal concentration) values by using the Microsoft Excel 2016.

### Results

### Liposomes Preparation and encapsulation of nucleoside analogues

2-(6-Amino-9H-purin-9-yl) quinazolin-4-ol and {[2-(6-amino-9H-purin-9-yl)quinazolin-4-yl]oxy}phosphonic acid were tested at different liposomal systems. At Table 3, the results of the encapsulation efficacy, as calculated according equation (1), of each system can be seen.

| **{[2-(6-amino-9H-purin-9-yl)quinazolin-4-yl]oxy} phosphonic acid** | | **2-(6-Amino-9H-purin-9-yl) quinazolin-4-ol** | |
|---|---|---|---|
| **Code** | **Encapsulation Efficacy (EE %)** | **Code** | **Encapsulation Efficacy (EE %)** |
| **1** | 60.8 | 1 | 55.2 |
| 2 | **76.8** | 2 | 55.2 |
| **3** | 50.0 | **3** | **65.1** |
| **4** | 60.8 | 4 | 54.4 |
| **5** | 70.4 | 5 | 65.6 |

Liposomal systems 2 and 3 exhibited the most successful results for {[2-(6-amino-9H-purin-9-yl)quinazolin-4-yl]oxy}phosphonic acid and 2-(6-Amino-9H-purin-9-yl) quinazolin-4-ol, respectively. These two systems (Liposomal systems 2 and 3) were tested for the rest of nucleoside analogues, and the encapsulation efficacy was calculated indirectly based on equation (1) and the standard curves (Figure 3).

For example, for {[2-(6-amino-9H-purin-9-yl)quinazolin-4-yl]oxy}phosphonic acid system code 1, after the centrifugation, we keep the supernatant from which, a fraction is collected to prepare a solution with targeted concertation of 10 ppm. Based on the Dilution Equation, C1V1=C2V2, C1=1000, because we consider, at the initial solution that the concentration is 1000 ppm (based on the fact that all of the added nucleoside analogue would be in the supernatant)

C2: is the targeted concentration, here 10ppm

V2: volume of the final solution, here 10 mL

Thus, we take 100uL from the supernatant and we dilute it to 10mL final volume. The sample is quantified by UV/VIS spectrometry and we calculate the actual concentration of the sample. The concentration is converted to mass and we can estimate the final mass of the nucleoside analogue in the supernatant and in the liposome.

Liposomal systems 2 and 3 were used to develop liposomes with the rest of the nucleoside analogues. The standard curves of the nucleoside analogues in PBS can be seen in Figure 3.

| **Encapsulation efficacy of nucleoside analogues encapsulated into liposomal systems 2 and 3** | | | |
|---|---|---|---|
| N-[1-(4-hydroxyquinazolin-2-yl)-2-oxo-1,2-dihydropyrimidin-4-yl]acetamide | | 4-amino-1-(4-hydroxyquinazolin-2-yl)-1,2-dihydropyrimidin-2-one | |
| **Liposomal system** | **Encapsulation Efficacy (EE %)** | **Liposomal system** | **Encapsulation Efficacy (EE %)** |
| 2 | 58.1 | 2 | 73.6 |
| 3 | 89.9 | 3 | 76.2 |

As it is seen from the results summarized in the Table above, the liposomal System 3 was found to display a good encapsulation efficiency and was used as the liposomal system for all nucleoside analogues, except for {[2-(6-amino-9H-purin-9-yl)quinazolin-4-yl]oxy}phosphonic acid, for which analogue System 2 was retained.

**Yields**

The yield of different liposomal systems was determined based on the mass of the product after the freeze drying cycle. The initial mass of each product is calculated by the lipids and the molecule added.

| **Nucleoside Analogue Liposome** | **Yield (%)** |
|---|---|
| N-[1-(4-hydroxyquinazolin-2-yl)-2-oxo-1,2-dihydropyrimidin-4-yl]acetamide * | 87.7 |
| 4-amino-1-(4-hydroxyquinazolin-2-yl)-1,2-dihydropyrimidin-2-one * | 89.3 |
| {[2-(6-amino-9H-purin-9-yl)quinazolin-4-yl]oxy}phosphonic acid $^+$ | 48.4 |

(continued)

| Nucleoside Analogue Liposome | Yield (%) |
|---|---|
| *2-(6-Amino-9H-purin-9-yl) quinazolin-4-ol** | 77.84 |
| **:in System 3 /+:in System 2* | |

## DSC

Blank liposomes and nucleoside-analogues encapsulated liposomes exhibit similar endo peaks at the beginning of the thermograph. All liposomes, whether loaded or blank, apart from the {[2-(6-amino-9H-purin-9-yl)quinazolin-4-yl]oxy}phosphonic acid liposome of thermograph (7), seem to have the same glass transition temperature, around 35°C.

There are characteristic endothermic peaks observed at liposomes in the range of 40°C to 100°C, in all cases, apart from the {[2-(6-amino-9H-purin-9-yl)quinazolin-4-yl]oxy}phosphonic acid liposomes. These peaks arise from phosphatidylcholine in these products which is not used in the case of {[2-(6-amino-9H-purin-9-yl)quinazolin-4-yl]oxy}phosphonic acid liposomes. That is why in this liposome, there is a different broad endothermic peak at the beginning of the thermograph. Furthermore, in the DSC thermographs, where the phase transition peaks are visible, it is apparent that since almost all of the liposomes had the same formulation, their peaks display significant changes. It is believed that these changes are attributable to the different chemical nature of each nucleoside analogue.

## SEM

Photographs of the freeze dried liposomes were taken and studied for their morphological properties as well as for their size. All of the liposomes seem to have the same appearance, almost spherical and uniform in size particles. Their size was approximately calculated with the use of the sizing tool of this instrument.

The SEM photographs in Figures 5, 6 and 7 show liposomes. The below Table reports liposome size as determined using the sizing tool on the SEM photographs.

| Product | Average Size (nm) | ±SD |
|---|---|---|
| Blank Liposome 1 (System 3) | 713 | 2 |
| Blank Liposome 2 (System 2) | 646.7 | 19.5 |
| Liposome {[2-(6-amino-9H-purin-9-yl)quinazolin-4-yl]oxy}phosphonic acid + | 820.3 | 15.95 |
| Liposome 2-(6-Amino-9H-purin-9-yl) quinazolin-4-ol* | 923.7 | 15.63 |
| Liposome N-[1-(4-hydroxyquinazolin-2-yl)-2-oxo-1,2-dihydropyrimidin-4-yl] acetamide * | 815.0 | 54.08 |
| Liposome 4-amino-1-(4-hydroxyquinazolin-2-yl)-1,2-dihydropyrimidin-2-one * | 847.3 | 14.05 |
| *:in System 3 / +: in System 2 | | |

Based on the above measurements, it can be demonstrated that liposomes encapsulating nucleoside analogues are significantly greater in size, showing that the encapsulation of an active substance inside a liposome can increase its size.

## In vitro assessment of cytotoxic activity

The below Table illustrates the cytotoxic effects of the five liposomes encapsulating nucleoside analogues against four human cancer cell lines. LC50 values were calculated for the conditions were at least 50 % of cell viability was observed in the range of the tested concentrations, 0.01μM to 10μM. 2-(6-Amino-9H-purin-9-yl) quinazolin-4-ol, when encapsulated in liposomes, or without being encapsulated in liposomes (not shown in Table) had no statistically significant cytotoxic effect against any human cancer cell lines and at any time points in the range of 0.01μM to 10μM. MDA-MB-231 human breast adenocarcinoma cell line exhibited resistance against all of the tested nucleoside analogues, when encapsulated in liposomes or without being encapsulated in liposomes (not shown in Table). For the remaining cancer cell lines, COLO699N, HCT116 and MCF7, no nucleoside analogue had a statistically significant cytotoxic effect when it was used alone (not shown in Table), i.e. without being encapsulated in liposomes. However, when encapsulated

in liposomes , for each cancer cell line, specific nucleoside analogues , when encapsulated in liposomes, exhibited significant inhibition of cell growth and in many cases the LC50 was lower than the minimum testing concentration (0.01μM). In particular, liposomes of 4-amino-1-(4-hydroxyquinazolin-2-yl)-1,2-dihydropyrimidin-2-one and of {[2-(6-amino-9H-purin-9-yl)quinazolin-4-yl]oxy}phosphonic acid in System 3 and 2, respectively, were highly effective in COLO699N, HCT116 and MCF7 at sub-micromole concentrations, as were liposomes of N-[1-(4-hydroxyquinazolin-2-yl)-2-oxo-1,2-dihydropyrimidin-4-yl]acetamide in System 3.

**Table.** LC50 values for the five liposomes encapsulating nucleoside analogues against four human cancer cell lines.

| **LC50** | Liposome N-[1-(4-hydroxyquinazol in-2-yl)-2-oxo-1,2-dihydropyrimidin -4-yl]acetamide | Liposome 4-amino-1-(4-hydroxyquinaz olin-2-yl)-1,2-dihydropyrimidi n-2-one | Liposome{[2-(6-amino-9H-purin-9-yl) quinazolin-4-yl]oxy} phospho nic acid | Liposome 2-(6-Amino-9H-purin-9-yl) quinazolin-4-ol |
|---|---|---|---|---|
| COLO699N | >10μM | **0.06μM (48h)** | **2.03μM (48h)** | >10μM |
| HCT116 | **4.06μM (48h)** | **<0.01μM (48h)**<br>**<0.01μM (72h)** | **<0.01μM (48h)**<br>**<0.01μM (72h)** | >10μM |
| MCF7 | >10μM | **<0.01μM (24h)**<br>**3.56μM (72h)** | **5.75μM (24h)** | >10μM |
| MDA-MB-231 | >10μM | >10μM | >10μM | >10μM |

## Claims

1. A pharmaceutical composition comprising a nucleoside analogue loaded into liposomes of a liposomal delivery system, wherein the nucleoside analogue is a pyrimidine analogue chosen from 4-amino-1-(4-hydroxyquinazolin-2-yl)-1,2-dihydropyrimidin-2-one or N-[1-(4-hydroxyquinazolin-2-yl)-2-oxo-1,2-dihydropyrimidin-4-yl]acetamide; or a purine analogue chosen from {[2-(6-amino-9H-purin-9-yl)quinazolin-4-yl]oxy}phosphonic acid or 2-(6-amino-9H-purin-9-yl) quinazolin-4-ol.

2. The pharmaceutical composition according to claim 1, wherein the nucleoside analogue is covalently coupled or is not covalently coupled to the liposome.

3. The pharmaceutical composition according to any preceding claim, wherein the liposomes of the liposomal delivery system have a diameter of at least 750, preferably of at least 775, and more preferably of at least 800 nm, as determined by SEM imaging.

4. The pharmaceutical composition according to any preceding claim, wherein the liposomes of the liposomal delivery system are sterically stabilized liposomes.

5. The pharmaceutical composition according to any preceding claim, wherein the liposomes of the liposomal delivery system comprise at least a phospholipid, preferably at least a phosphatidylcholine and/or a phosphoethanolamine, or comprise at least a phospholipid, more preferably at least a phosphatidylcholine and/or a phosphoethanolamine, most preferably in addition to one or more phosphogylcerides and/or one or more sphingolipids.

6. The pharmaceutical composition according to any preceding claim, wherein the liposomes of the liposomal delivery system comprise, or essentially consist of, as the lipid component(s) of the liposomes, a pegylated phospholipid such as pegylated 1,2-distearoyl-sn-glycero-3-phosphoethanolamine, or a combination of a peglyated and an unpegylated phospholipid such as such as pegylated 1,2-distearoyl-sn-glycero-3-phosphoethanolamine and unpegylated phosphatidylcholine, respectively, optionally together with a sterol such as cholesterol.

7. The pharmaceutical composition according to claim 6, wherein the pegylated phospholipid is pegylated phosphoethanolamine and the unpegylated phospholipid is a phosphatidylcholine.

8. The pharmaceutical composition according to any preceding claim, wherein the liposomes of the liposomal delivery

system are free of fatty acids, in particular free of unsaturated fatty acids, and preferably of monounsaturated fatty acids such as oleic acid.

**9.** The pharmaceutical composition according to any preceding claim, wherein the liposomes of the liposomal delivery system comprise an amount of sterol such as cholesterol of from about more than 5 wt% to less than 50 wt%, preferably of from about more than 5 wt% to less than 25 wt%, more preferably of from about more than 5 wt% to less than 20 wt%, most preferably of from about more than 6 wt% to less than 16 wt%, based on the weight of the pegylated phospholipid or the weight of the combination of a peglyated and an unpegylated phospholipid.

**10.** The pharmaceutical composition according to any preceding claim, wherein the liposomes of the liposomal delivery system comprises, or consists of, of from 40 to 45 wt% of a pegylated phospholipid such as N-(carbonyl-methoxypolyethylene glycol-2000)-1,2-distearoyl-sn-glycero-3 phosphoethanolamine, of from 40 to 45 wt% of an unpegylated phospholipid such as phosphatidylcholine, and of a sterol such as cholesterol, based on the total weight of the pegylated phospholipid, unpegylated phospholipid and sterol, and preferably wherein the pegylated phospholipid and unpegylated phospholipid are comprised at a weight ratio of 1:1.

**11.** A pharmaceutical composition according to any preceding claim for use in the treatment of colon cancer or lung cancer or breast cancer, preferably via oral or parenteral route.

**12.** Use of a pharmaceutical composition according to any preceding claim for the inhibition of proliferation of cancer cell lines *in vitro,* preferably in a concentration of 0.01$\mu$M to 10$\mu$M

**13.** The use according to claim 12 wherein the cancer cell line is a lung or colon or breast cancer cell line and the nucleoside analog is 4-amino-1-(4-hydroxyquinazolin-2-yl)-1,2-dihydropyrimidin-2-one or {[2-(6-amino-9H-purin-9-yl)quinazolin-4-yl]oxy}phosphonic acid, and preferably the nucleoside analog is present in a concentration of 0.01$\mu$M to 1$\mu$M.

**14.** A process for producing a liposomal delivery system comprising a nucleoside analogue loaded into liposomes,

wherein the liposomes of the liposomal delivery system comprise, or consist of, a combination of a pegylated and an unpegylated phospholipid, and preferably of from 40 to 45 wt% of a pegylated phospholipid such as N-(carbonyl-methoxypolyethylene glycol-2000)-1,2-distearoyl-sn-glycero-3 phosphoethanolamine, of from 40 to 45 wt% of an unpegylated phospholipid such as phosphatidylcholine, and of a sterol such as cholesterol, based on the total weight of the pegylated phospholipid, unpegylated phospholipid and sterol, and more preferably wherein the pegylated phospholipid and unpegylated phospholipid are comprised at a weight ratio of 1:1, and
wherein the nucleoside analogue is chosen among 4-amino-1-(4-hydroxyquinazolin-2-yl)-1,2-dihydropyrimidin-2-one, or N-[1-(4-hydroxyquinazolin-2-yl)-2-oxo-1,2-dihydropyrimidin-4-yl]acetamide,
wherein the process comprises the steps of:

a. solubilizing the pegylated phospholipid, unpegylated phospholipid, and sterol, and the nucleoside analogue, in an polar solvent, preferably in a mixture of polar solvents,
b. evaporating the solvent, and
c. adding an aqueous solution or water, preferably an aqueous buffer solution, such as to form a first liposomal suspension , and
d. sonicating the first liposomal suspension to form a second liposomal suspension,
e. annealing the second liposomal suspension to form the liposomal delivery system comprising a nucleoside analogue loaded into liposomes.

**15.** A process for producing a liposomal delivery system comprising a nucleoside analogue loaded into liposomes,

wherein the liposomes of the liposomal delivery system comprise, or consist of, a combination of a pegylated and an unpegylated phospholipid, and preferably of from 40 to 45 wt% of a pegylated phospholipid such as N-(carbonyl-methoxypolyethylene glycol-2000)-1,2-distearoyl-sn-glycero-3 phosphoethanolamine, of from 40 to 45 wt% of an unpegylated phospholipid such as phosphatidylcholine, and of a sterol such as cholesterol, based on the total weight of the pegylated phospholipid, unpegylated phospholipid and sterol, and more preferably wherein the pegylated phospholipid and unpegylated phospholipid are comprised at a weight ratio of 1:1, and

wherein the nucleoside analogue is chosen among {[2-(6-amino-9H-purin-9-yl)quinazolin-4-yl]oxy}phosphonic acid and 2-(6-Amino-9H-purin-9-yl) quinazolin-4-ol,
wherein the process comprises the steps of:

a. solubilizing the pegylated phospholipid, unpegylated phospholipid, and sterol, in an polar solvent, preferably in a mixture of polar solvents,
b. evaporating the solvent, and
c. adding an aqueous solution of the nucleoside analogue , preferably an aqueous buffer solution of the nucleoside analogue, such as to form a first liposomal suspension, and
d. sonicating the first liposomal suspension to form a second liposomal suspension,
e. annealing the second liposomal suspension to form the liposomal delivery system comprising a nucleoside analogue loaded into liposomes.

**Patentansprüche**

1. Pharmazeutische Zusammensetzung, aufweisend ein Nukleosidanalog, welches in Liposome eines liposomalen Zuführsystems hinein geladen ist, wobei das Nukleosidanalog ein Pyrimidinanalog ist, welches ausgewählt ist aus 4-Amino-1-(4-Hydroxyquinazolin-2-yl)-1,2-Dihydropyrimidin-2-on oder N-[1-(4-Hydroxyquinazolin-2-yl)-2-oxo-1,2-Dihydropyrimidin-4-yl]Acetamid; oder ein Purinanalog, ausgewählt aus {[2-(6-Amino-9H-Purin-9-yl)Quinazolin-4-yl]oxy}Phosphonsäure oder 2-(6-Amino-9H-Purin-9-yl)Quinazolin-4-ol.

2. Pharmazeutische Zusammensetzung gemäss Anspruch 1, wobei das Nukleosidanalog an das Liposom kovalent gekoppelt oder nicht kovalent gekoppelt ist.

3. Pharmazeutische Zusammensetzung gemäss einem der vorhergehenden Ansprüche, wobei die Liposomen des liposomalen Zuführsystems einen Durchmesser von mindestens 750, vorzugsweise von mindestens 775 und insbesondere bevorzugt von mindestens 800 nm aufweisen, wie durch REM Bildgebung bestimmt.

4. Pharmazeutische Zusammensetzung gemäss einem der vorhergehenden Ansprüche, wobei die Liposomen des liposomalen Zuführsystems sterisch stabilisierte Liposomen sind.

5. Pharmazeutische Zusammensetzung gemäss einem der vorhergehenden Ansprüche, wobei die Liposomen des liposomalen Zuführsystems mindestens ein Phospholipid aufweisen, vorzugsweise mindestens ein Phosphatidylcholin und/oder ein Phosphoethanolamin, oder mindestens ein Phospholipid aufweisen, insbesondere bevorzugt mindestens ein Phosphatidylcholin und/oder ein Phosphoethanolamin, am meisten bevorzugt zusätzlich zu einem oder mehreren Phosphoglyceriden und/oder einem oder mehreren Sphingolipiden.

6. Pharmazeutische Zusammensetzung gemäss einem der vorhergehenden Ansprüche, wobei die Liposomen des liposomalen Zuführsystems als die Lipidkomponente(n) der Liposomen ein pegyliertes Phospholipid, wie beispielsweise pegyliertes 1,2-Distearoyl-sn-Glycero-3-Phosphoethanolamin aufweisen, oder im Wesentlichen daraus bestehen, oder eine Kombination eines pegylierten Phospholipidsund eines nicht-pegylierten Phospholipids aufweisen, oder im Wesentlichen daraus bestehen, wie beispielsweise pegyliertes 1,2-Distearoyl-sn-Glycero-3-Phosphoethanolamin bzw. unpegyliertes Phosphatidylcholin, optional zusammen mit einem Sterol, wie beispielsweise Cholesterin.

7. Pharmazeutische Zusammensetzung gemäss Anspruch 6, wobei das pegylierte Phospholipid pegyliertes Phosphoethanolamin ist und das nicht-pegylierte Phospholipid ein Phosphatidylcholin ist.

8. Pharmazeutische Zusammensetzung gemäss einem der vorhergehenden Ansprüche, wobei die Liposomen des liposomalen Zuführsystems frei von Fettsäuren sind, insbesondere frei von ungesättigten Fettsäuren, und vorzugsweise von einfachungesättigten Fettsäuren, wie beispielsweise Ölsäure.

9. Pharmazeutische Zusammensetzung gemäss einem der vorhergehenden Ansprüche, wobei die Liposomen des liposomalen Zuführsystems eine Menge an Sterol aufweisen, beispielsweise Cholesterin mit etwa mehr als 5 Gewichtsprozent bis weniger als 50 Gewichtsprozent, vorzugsweise von etwa mehr als 5 Gewichtsprozent bis weniger als 25 Gewichtsprozent, insbesondere bevorzugt von etwa mehr als 5 Gewichtsprozent bis weniger als 20 Gewichtsprozent, am meisten bevorzugt von etwa mehr als 6 Gewichtsprozent bis weniger als 16 Gewichtsprozent,

basierend auf dem Gewicht des pegylierten Phospholipids oder dem Gewicht der Kombination eines pegylierten und eines nicht-pegylierten Phospholipids.

**10.** Pharmazeutische Zusammensetzung gemäss einem der vorhergehenden Ansprüche, wobei die Liposomen des liposomalen Zuführsystems von 40 bis 45 Gewichtsprozent eines pegylierten Phospholipids, wie beispielsweise N-(Carbonyl-Methoxypolyethylenglycol-2000)-1,2-Distearoyl-sn-Glycero-3-Phosphoethanolamin, von zwischen 40 bis 45 Gewichtsprozent eines nicht-pegylierten Phospholipids, wie beispielsweise Phosphatidylcholin, und ein Sterol, wie beispielsweise Cholesterin, aufweisen oder daraus bestehen, basierend auf dem Gesamtgewicht des pegylierten Phospholipids, nicht-pegylierten Phospholipids und Sterols, und wobei vorzugsweise das pegylierte Phospholipid und das nicht-pegylierte Phospholipid in einem Gewichtsverhältnis von 1:1 enthalten sind.

**11.** Pharmazeutische Zusammensetzung gemäss einem der vorhergehenden Ansprüche, zur Verwendung in der Behandlung von Darmkrebs oder Lungenkrebs oder Brustkrebs, vorzugsweise über orale oder parenterale Aufnahme.

**12.** Verwendung einer pharmazeutischen Zusammensetzung gemäss einem der vorhergehenden Ansprüche zur Hemmung der Vermehrung von Krebszelllinien *in vitro,* vorzugsweise in einer Konzentration von 0.01 $\mu$M bis 10 $\mu$M.

**13.** Verwendung gemäss Anspruch 12, wobei die Krebszelllinie eine Lungen- oder Darm- oder Brustkrebszelllinie ist und das Nukleosidanalog ein 4-Amino-1-(4-Hydroxyquinazolin-2-yl)-1,2-Dihydropyrimidin-2-on oder {[2-(6-Amino-9H-Purin-9-yl)Quinazolin-4-yl]oxy}Phosphonsäure ist, und vorzugsweise das Nukleosidanalog in einer Konzentration von 0.01 $\mu$M bis 1 $\mu$M vorliegt.

**14.** Verfahren zur Herstellung eines liposomalen Zuführsystems, aufweisend ein Nukleosidanalog, welches in Liposome hinein geladen ist,
wobei die Liposomen des liposomalen Zuführsystems eine Kombination eines pegylierten und eines nicht-pegylierten Phospholipids aufweisen oder daraus bestehen, vorzugsweise von 40 bis 45 Gewichtsprozent eines pegylierten Phospholipids, wie beispielsweise N-(Carbonyl-Methoxypolyethylenglycol-2000)-1,2-Distearoyl-sn-Glycero-3-Phosphoethanolamin, von 40 bis 45 Gewichtsprozent eines nicht-pegylierten Phospholipids, wie beispielsweise Phosphatidylcholin, und eines Sterols, wie beispielsweise Cholesterin, basierend auf dem Gesamtgewicht des pegylierten Phospholipids, nicht-pegylierten Phospholipids, und Sterols, und wobei insbesondere bevorzugt das pegylierte Phospholipid und nicht-pegylierte Phospholipid in einem Gewichtsverhältnis von 1:1 enthalten sind, und wobei das Nukleosidanalog ausgewählt ist aus 4-Amino-1-(4-Hydroxyquinazolin-2-yl)-1,2-Dihydropyrimidin-2-on, oder N-[1-(4-Hydroxyquinazolin-2-yl)-2-oxo-1,2-Dihydropyrimidin-4-yl]Acetamid, wobei das Verfahren die folgenden Schritte beinhaltet:

a. Solubilisieren des pegylierten Phospholipids, nicht-pegylierten Phospholipids, und Sterols, und des Nucleosidanalogs, in einem polaren Lösungsmittel, vorzugsweise in einer Mischung aus polaren Lösungsmitteln,
b. Verdampfen des Lösungsmittels, und
c. Beifügen von einer wässrigen Lösung oder von Wasser, vorzugsweise von einer wässrigen Pufferlösung, zum Bilden einer ersten liposomalen Suspension, und
d. Behandeln der ersten liposomalen Suspension im Ultraschallbad, zum Bilden einer zweiten liposomalen Suspension,
e. Ausheilen der zweiten liposomalen Suspension, zum Bilden des liposomalen Zuführsystems, aufweisend ein Nukleosidanalog, welches in Liposome hinein geladen ist.

**15.** Verfahren zur Herstellung eines liposomalen Zuführsystems, aufweisend ein Nukleosidanalog, welches in Liposome hinein geladen ist,
wobei die Liposomen des liposomalen Zuführsystems eine Kombination von einem pegylierten und einem nicht-pegylierten Phospholipid aufweisen oder daraus bestehen, und vorzugsweise von 40 bis 45 Gewichtsprozent eines pegylierten Phospholipids, wie beispielsweise N-(Carbonyl-Methoxypolyethylenglycol-2000)-1,2-Distearoyl-sn-Glycero-3-Phosphoethanolamin, von 40 bis 45 Gewichtsprozent eines nicht-pegylierten Phospholipids, wie beispielsweise Phosphatidylcholin, und von einem Sterol, wie beispielsweise Cholesterin, basierend auf dem Gesamtgewicht des pegylierten Phospholipids, nicht-pegylierten Phospholipids und Sterols, und wobei insbesondere bevorzugt das pegylierte Phospholipid und nicht-pegylierte Phospholipid in einem Gewichtsverhältnis von 1:1 enthalten sind, und wobei das Nukleosidanalog ausgewählt ist aus {[2-(6-Amino-9H-Purin-9-yl)Quinazolin-4-yl]oxy}Phosphonsäure und 2-(6-Amino-9H-Purin-9-yl)Quinazolin-4-ol, wobei das Verfahren die folgenden Schritte beinhaltet:

a. Solubilisieren des pegylierten Phospholipids, nicht-pegylierten Phospholipids, und Sterols, in einem polaren

Lösungsmittel, vorzugsweise in einer Mischung von polaren Lösungsmitteln,
b. Verdampfen des Lösungsmittels, und
c. Beifügen von einer wässrigen Lösung des Nukleosidanalogs, vorzugsweise von einer wässrigen Pufferlösung des Nukleosidanalogs, zum Bilden einer ersten liposomalen Suspension, und
d. Behandeln der ersten liposomalen Suspension im Ultraschallbad, zum Bilden einer zweiten liposomalen Suspension,
e. Ausheilen der zweiten liposomalen Suspension, zum Bilden des liposomalen Zuführsystems, aufweisend ein Nukleosidanalog, welches in Liposome hinein geladen ist.

## Revendications

1. Une composition pharmaceutique comprenant un analogue de nucléoside chargé dans des liposomes d'un système de distribution liposomal, dans laquelle l'analogue de nucléoside est un analogue de pyrimidine choisi parmi le 4-amino-1-(4-hydroxyquinazoline-2-yl)-1,2-dihydropyrimidine-2-one ou N-[1-(4-hydroxyquinazoline-2-yl)-2-oxo-1,2-dihydropyrimidine-4-yl]acétamide; ou un analogue de purine choisi parmi l'acide {[2-(6-amino-9H-purine-9-yl)quinazoline-4-yl]oxy}phosphonique ou 2-(6-amino-9H-purine-9-yl)quinazoline -4-ol.

2. La composition pharmaceutique selon la revendication 1, dans laquelle l'analogue de nucléoside est couplé de manière covalente au liposome ou n'est pas couplé de manière covalente au liposome.

3. La composition pharmaceutique selon l'une quelconque des revendications précédentes, dans laquelle les liposomes du système de distribution liposomal ont un diamètre d'au moins 750 nm, de préférence d'au moins 775 nm, et plus préférablement d'au moins 800 nm, tel que déterminé par imagerie SEM.

4. La composition pharmaceutique selon l'une quelconque des revendications précédentes, dans laquelle les liposomes du système de distribution liposomal sont des liposomes stériquement stabilisés.

5. La composition pharmaceutique selon l'une quelconque des revendications précédentes, dans laquelle les liposomes du système de distribution liposomal comprennent au moins un phospholipide, de préférence au moins une phosphatidylcholine et/ou une phosphoéthanolamine, ou comprennent au moins un phospholipide, plus préférablement au moins une phosphatidylcholine et/ou une phosphoéthanolamine, de préférence en plus d'un ou plusieurs phosphoglycérides et/ou d'un ou plusieurs sphingolipides.

6. La composition pharmaceutique selon l'une quelconque des revendications précédentes, dans laquelle les liposomes du système de distribution liposomal comprennent, ou sont essentiellement constitués, comme composant(s) lipidique(s) des liposomes, un phospholipide pégylé tel que la 1,2-distéaroyl-sn-glycéro-3-phosphoéthanolamine pégylée, ou une combinaison d'un phospholipide pégylé et d'un phospholipide non pégylé tel que la 1,2-distéaroyl-sn-glycéro-3-phosphoéthanolamine pégylée et la phosphatidylcholine non pégylée, respectivement, éventuellement conjointement avec un stérol tel que le cholestérol.

7. La composition pharmaceutique selon la revendication 6, dans laquelle le phospholipide pégylé est la phosphoéthanolamine pégylée et le phospholipide non pégylé est une phosphatidylcholine.

8. La composition pharmaceutique selon l'une quelconque des revendications précédentes, dans laquelle les liposomes du système de distribution liposomal sont exempts d'acides gras, en particulier exempts d'acides gras insaturés, et de préférence d'acides gras monoinsaturés tels que l'acide oléique.

9. La composition pharmaceutique selon l'une quelconque des revendications précédentes, dans laquelle les liposomes du système de distribution liposomal comprennent une quantité de stérol tel que le cholestérol allant d'environ plus de 5 % en poids à moins de 50 % en poids, de préférence d'environ plus de 5 % en poids à moins de 25 % en poids, plus préférablement d'environ plus de 5 % en poids à moins de 20 % en poids, de préférence encore d'environ plus de 6 % en poids à moins de 16 % en poids, sur la base du poids du phospholipide pégylé ou du poids de la combinaison d'un phospholipide pégylé et d'un phospholipide non pégylé.

10. La composition pharmaceutique selon l'une quelconque des revendications précédentes, dans laquelle les liposomes du système de distribution liposomal comprennent, ou sont constitués de, 40 à 45 % en poids d'un phospholipide pégylé tel que le N-(carbonyl-méthoxypolyéthylène glycol-2000)-1,2-distéaroyl-sn-glycéro-3 phosphoéthanolamine,

de 40 à 45 % en poids d'un phospholipide non pégylé tel que la phosphatidylcholine, et d'un stérol tel que le cholestérol, sur la base du poids total du phospholipide pégylé, du phospholipide non pégylé et du stérol, et de préférence dans lequel le phospholipide pégylé et le phospholipide non pégylé sont compris dans un rapport de poids de 1:1.

**11.** La composition pharmaceutique selon l'une quelconque des revendications précédentes destinée à être utilisée dans le traitement du cancer du côlon ou du cancer du poumon ou du sein, de préférence par voie orale ou parentérale.

**12.** Utilisation d'une composition pharmaceutique selon l'une quelconque des revendications précédentes pour l'inhibition de la prolifération de lignées de cellules cancéreuses *in vitro,* de préférence à une concentration de 0,01 μM à 10 μM.

**13.** Utilisation selon la revendication 12, dans laquelle la lignée cellulaire cancéreuse est une lignée cellulaire cancéreuse du poumon, du côlon ou du sein et l'analogue de nucléoside est le 4-amino-1-(4-hydroxyquinazoline-2-yl)-1,2-dihydropyrimidine-2-one ou acide {[2-(6-amino-9H-purine-9-yl)quinazoline-4-yl]oxy}phosphonique, et de préférence l'analogue de nucléoside est présent à une concentration de 0,01 μM à 1 μM.

**14.** Un procédé pour produire un système de distribution liposomal comprenant un analogue de nucléoside chargé dans des liposomes,

dans lequel les liposomes du système de distribution liposomal comprennent, ou sont constitués d'une combinaison d'un phospholipide pégylé et non pégylé, et de préférence de 40 à 45 % en poids d'un phospholipide pégylé tel que la N-(carbonyle-méthoxypolyéthylèneglycol-2000)- 1,2-distéaroyl-sn-glycéro-3 phosphoéthanolamine, de 40 à 45 % en poids d'un phospholipide non pégylé tel que la phosphatidylcholine, et d'un stérol tel que le cholestérol, sur la base du poids total du phospholipide pégylé, du phospholipide non pégylé et du stérol , et plus préférablement dans lequel le phospholipide pégylé et le phospholipide non pégylé sont compris dans un rapport pondéral de 1:1, et dans lequel l'analogue de nucléoside est choisi parmi la 4-amino-1-(4-hydroxyquinazoline-2-yl)-1,2-dihydropyrimidine-2-one ou la N-[1-(4-hydroxyquinazoline-2-yl)-2-oxo-1,2-dihydropyrimidine-4-yl]acétamide, dans lequel le processus comprend les étapes suivantes :

a. solubiliser le phospholipide pégylé, le phospholipide non pégylé et le stérol, ainsi que l'analogue nucléosidique, dans un solvant polaire, de préférence dans un mélange de solvants polaires,
b. évaporer le solvant, et
c. ajouter une solution aqueuse ou de l'eau, de préférence une solution tampon aqueuse, de manière à former une première suspension liposomale, et
d. soniquer la première suspension liposomale pour former une seconde suspension liposomale,
e. recuire la seconde suspension liposomale pour former le système de distribution liposomal comprenant un analogue de nucléoside chargé dans des liposomes.

**15.** Un procédé pour produire un système de distribution liposomal comprenant un analogue de nucléoside chargé dans des liposomes,

dans lequel les liposomes du système de distribution liposomal comprennent, ou sont constitués d'une combinaison d'un phospholipide pégylé et non pégylé, et de préférence de 40 à 45 % en poids d'un phospholipide pégylé tel que la N-(carbonyle-méthoxypolyéthylèneglycol-2000)- 1,2-distéaroyl-sn-glycéro-3 phosphoéthanolamine, de 40 à 45 % en poids d'un phospholipide non pégylé tel que la phosphatidylcholine, et d'un stérol tel que le cholestérol, sur la base du poids total du phospholipide pégylé, du phospholipide non pégylé et du stérol, et plus préférablement dans lequel le phospholipide pégylé et le phospholipide non pégylé sont compris dans un rapport de poids de 1:1, et

dans lequel l'analogue de nucléoside est choisi parmi l'acide {[2-(6-amino-9H-purine-9-yl)quinazoline-4-yl]oxy} phosphonique et le 2-(6-amino-9H-purine-9-yl) quinazoline-4-ol,

dans lequel le processus comprend les étapes suivantes :

a. solubiliser le phospholipide pégylé, le phospholipide non pégylé et le stérol, dans un solvant polaire, de préférence dans un mélange de solvants polaires,
b. évaporer le solvant, et
c. ajouter une solution aqueuse de l'analogue de nucléoside, de préférence une solution tampon aqueuse de l'analogue de nucléoside, de manière à former une première suspension liposomale, et
d. la soniquer la première suspension liposomale pour former une seconde suspension liposomale,

e. recuire la seconde suspension liposomale pour former le système de distribution liposomal comprenant un analogue de nucléoside chargé dans des liposomes.

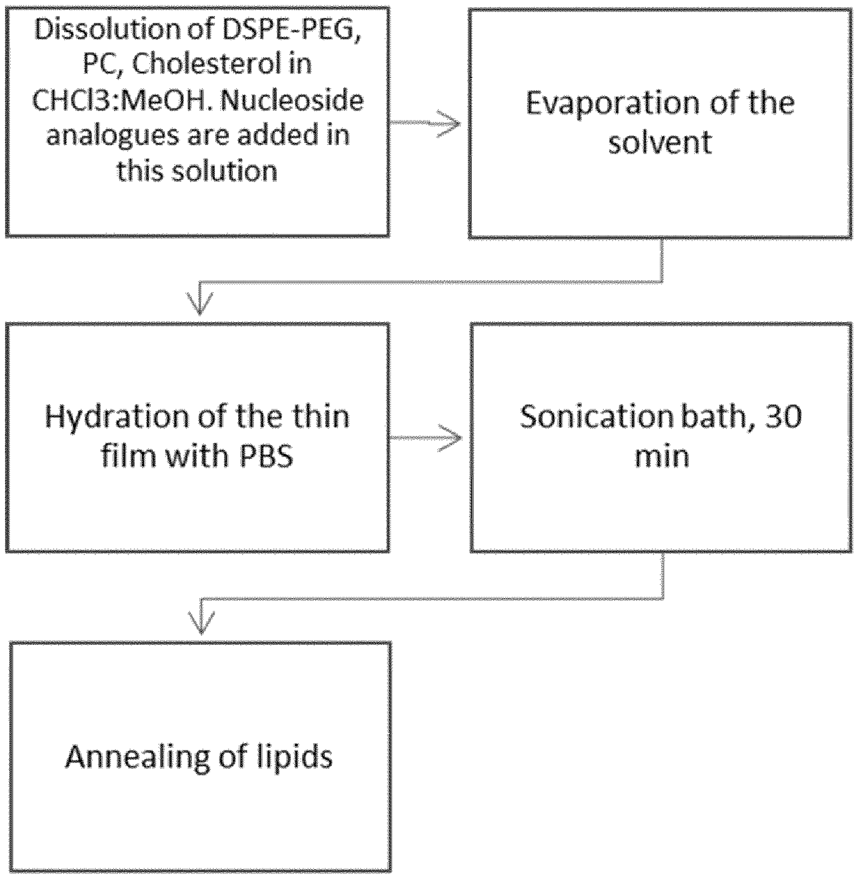
Dissolution of DSPE-PEG, PC, Cholesterol in CHCl3:MeOH. Nucleoside analogues are added in this solution
Evaporation of the solvent
Hydration of the thin film with PBS
Sonication bath, 30 min
Annealing of lipids

FIG. 1

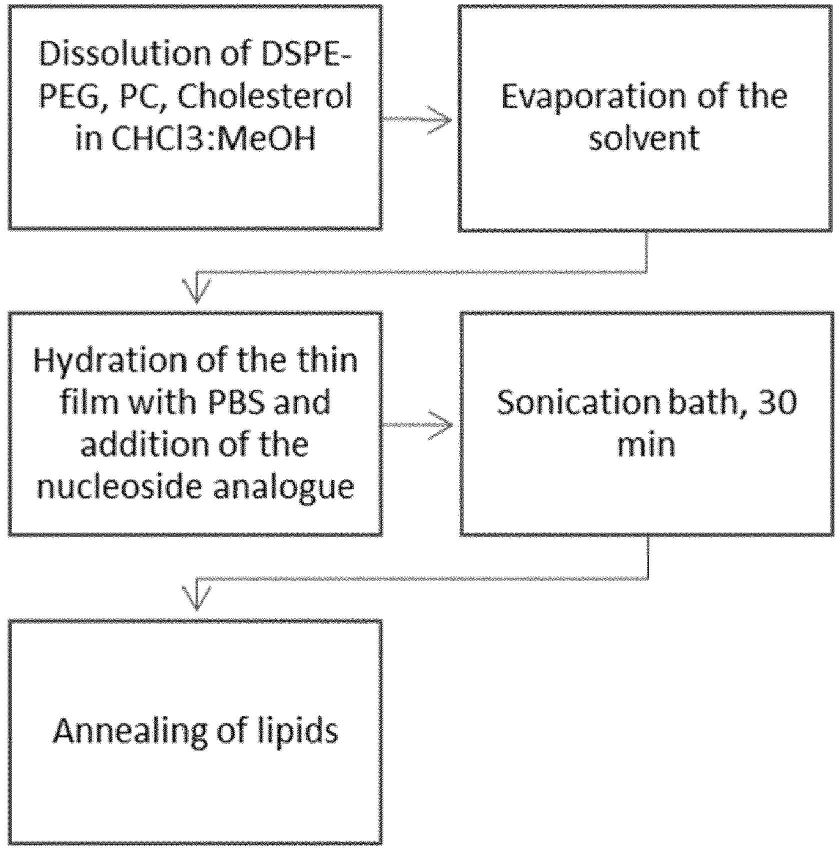
Dissolution of DSPE-PEG, PC, Cholesterol in CHCl3:MeOH
Evaporation of the solvent
Hydration of the thin film with PBS and addition of the nucleoside analogue
Sonication bath, 30 min
Annealing of lipids

FIG. 2

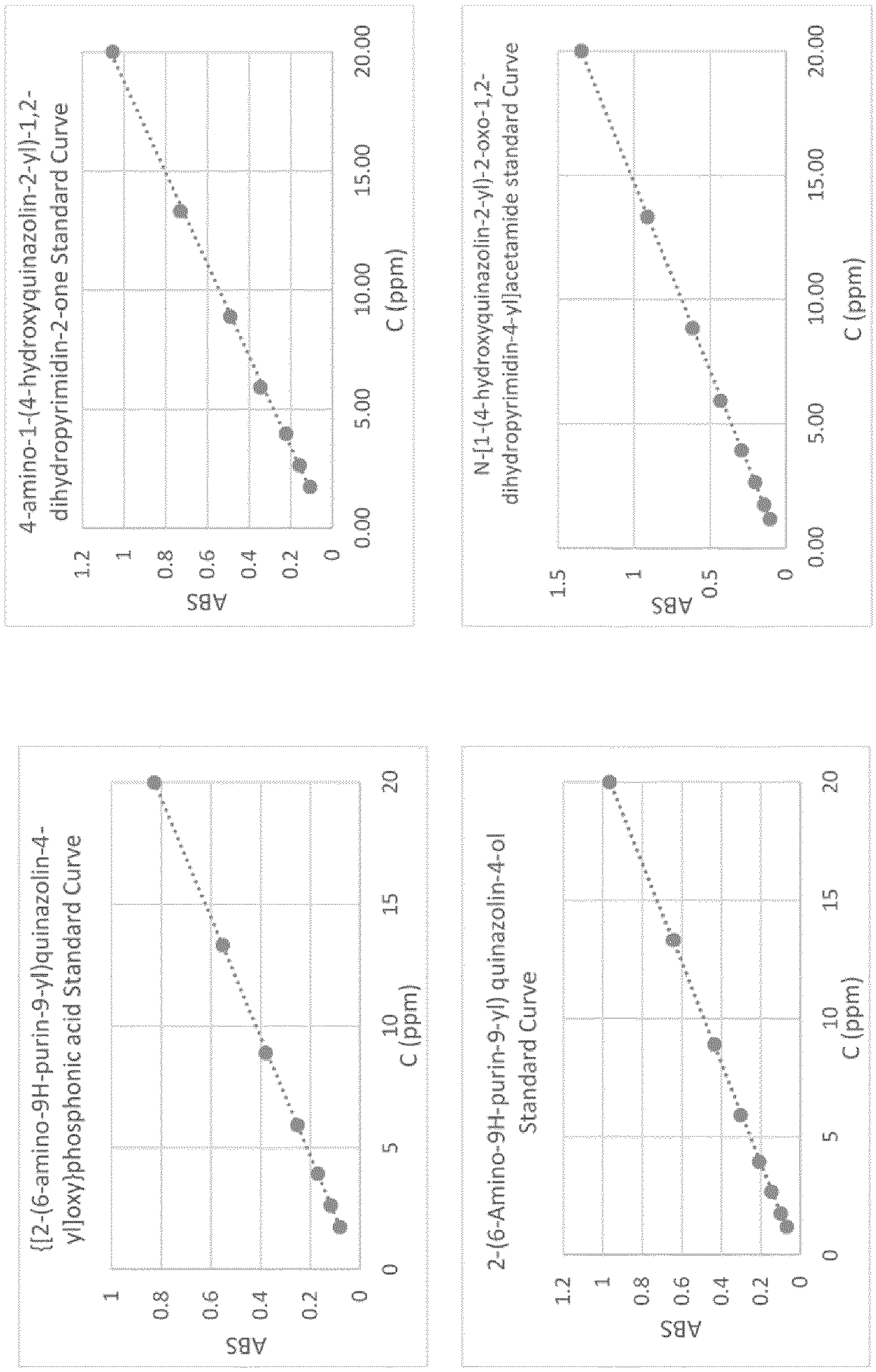
{[2-(6-amino-9H-purin-9-yl)quinazolin-4-yl]oxy}phosphonic acid Standard Curve
ABS
0
0.2
0.4
0.6
0.8
1
0
5
10
15
20
C (ppm)
4-amino-1-(4-hydroxyquinazolin-2-yl)-1,2-dihydropyrimidin-2-one Standard Curve
ABS
0
0.2
0.4
0.6
0.8
1
1.2
0.00
5.00
10.00
15.00
20.00
C (ppm)
2-(6-Amino-9H-purin-9-yl) quinazolin-4-ol Standard Curve
ABS
0
0.2
0.4
0.6
0.8
1
1.2
0
5
10
15
20
C (ppm)
N-[1-(4-hydroxyquinazolin-2-yl)-2-oxo-1,2-dihydropyrimidin-4-yl]acetamide standard Curve
ABS
0
0.5
1
1.5
0.00
5.00
10.00
15.00
20.00
C (ppm)

FIG. 3

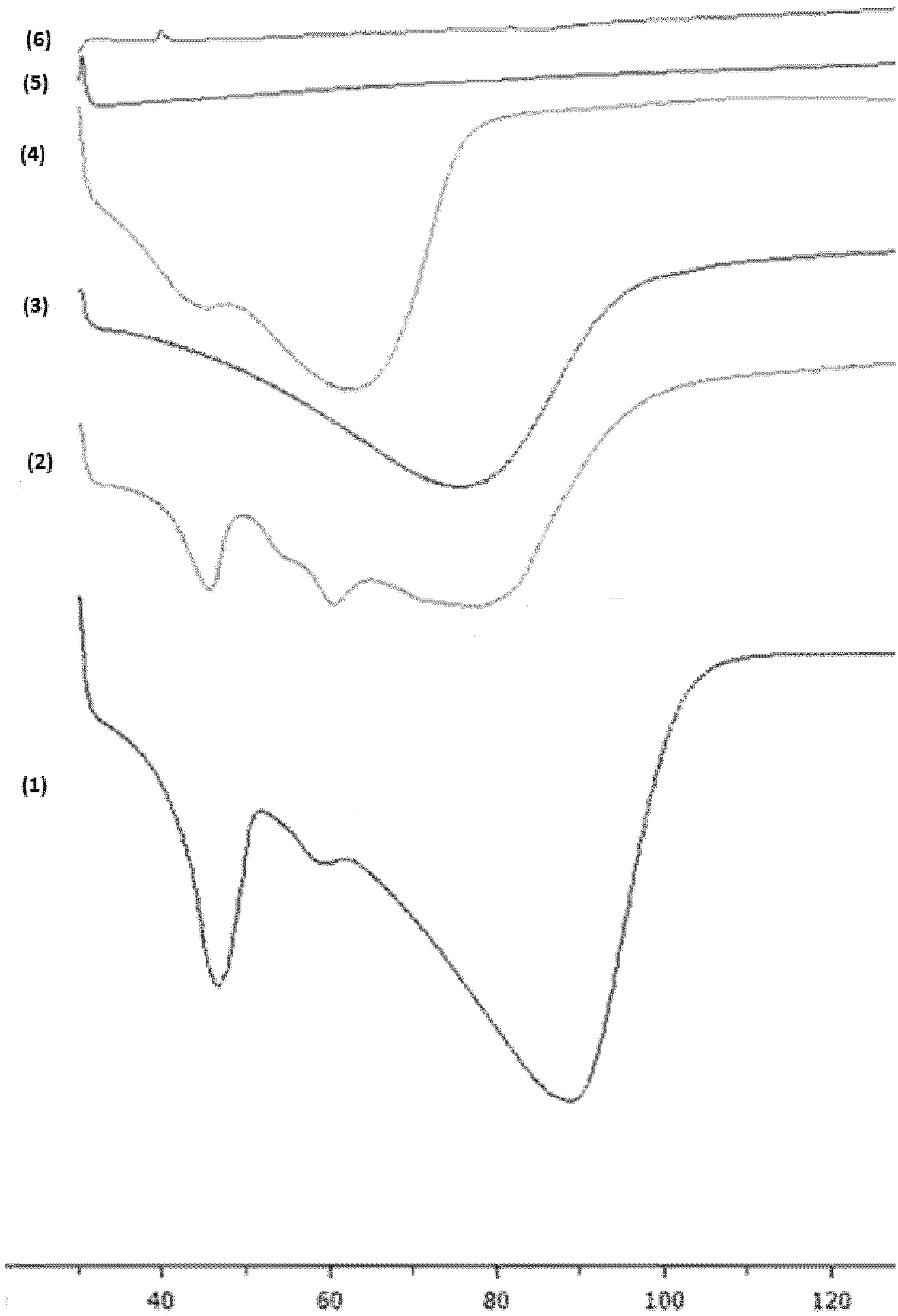


FIG. 4

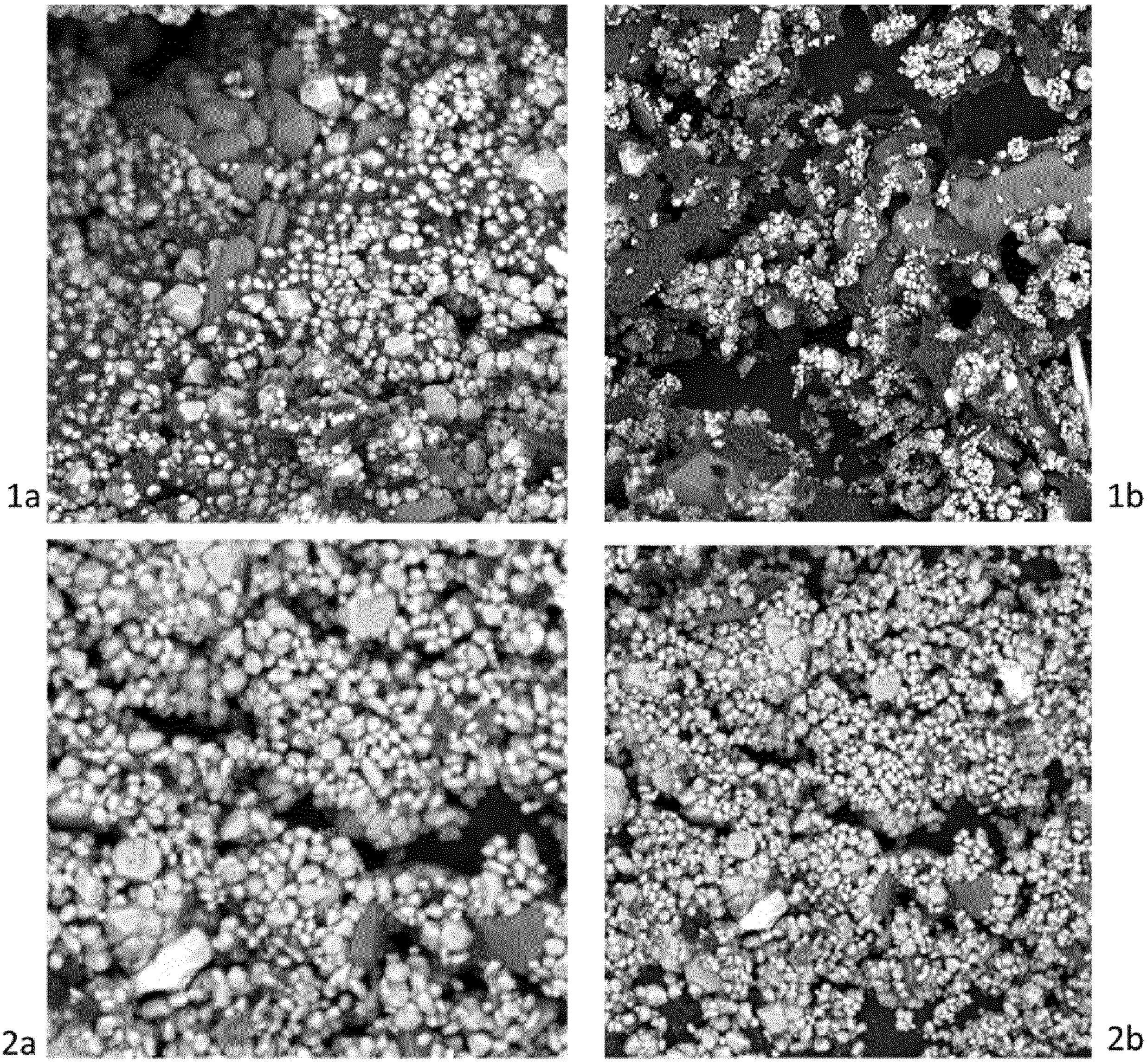


FIG. 5

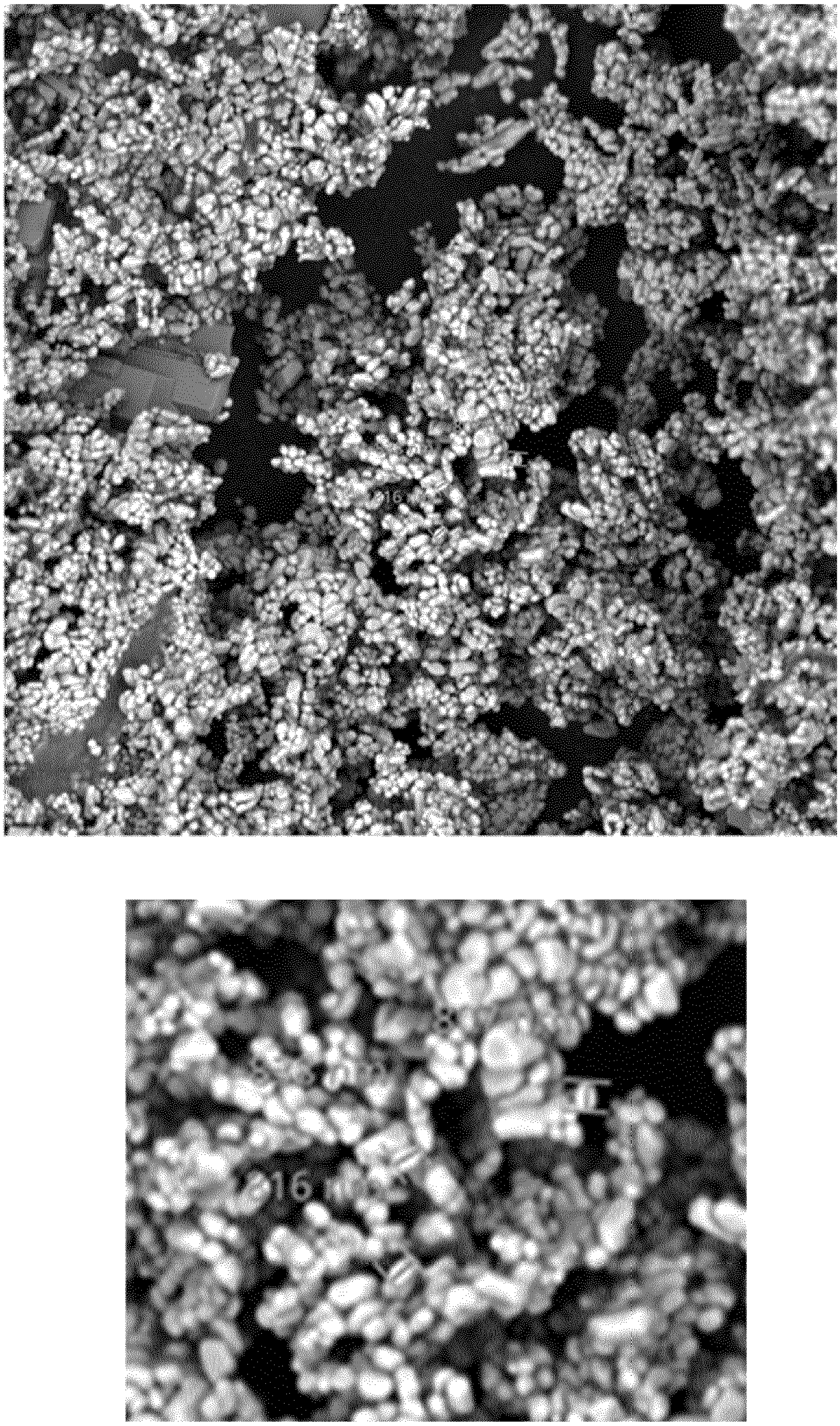

FIG. 6

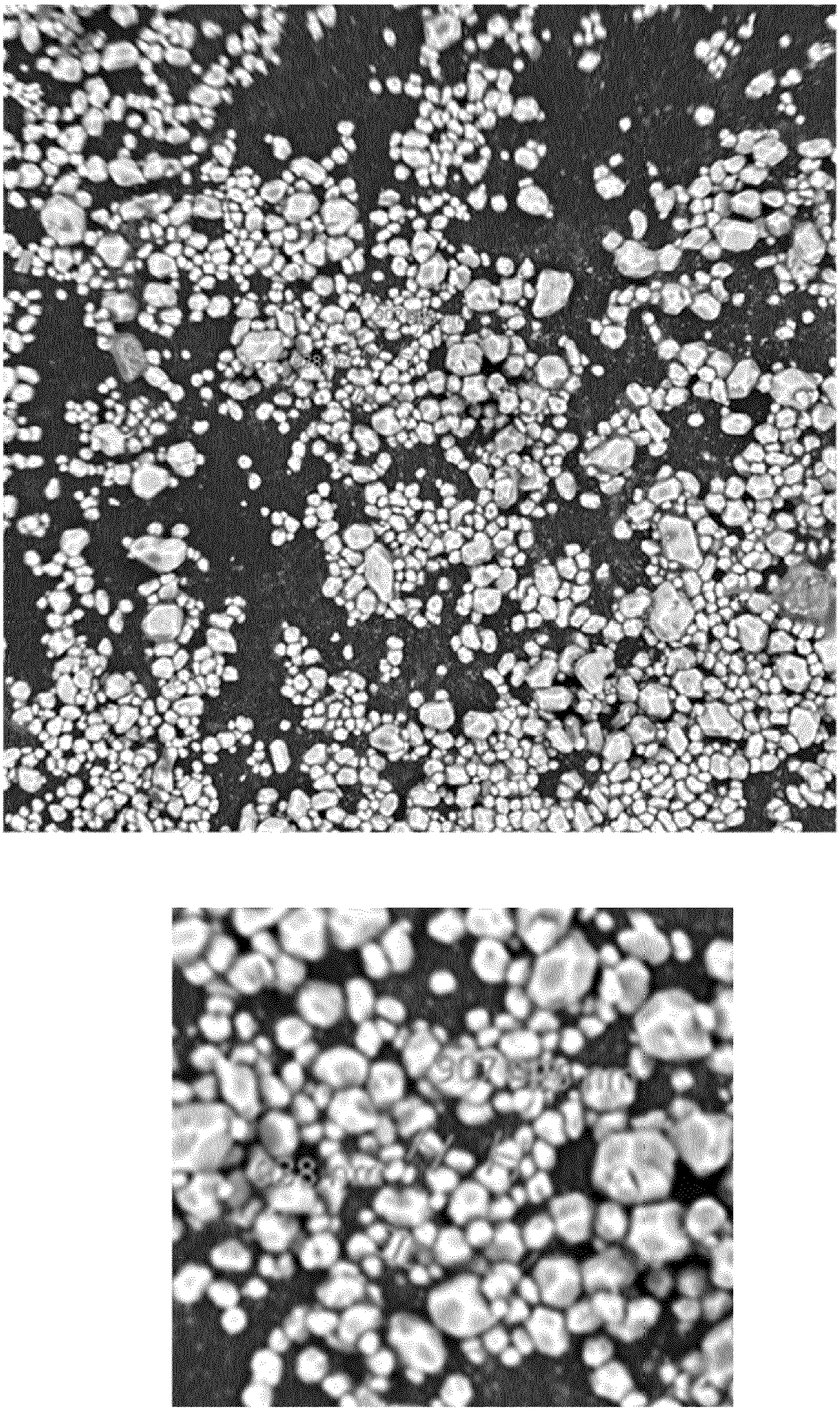

A

B

**FIG. 7**

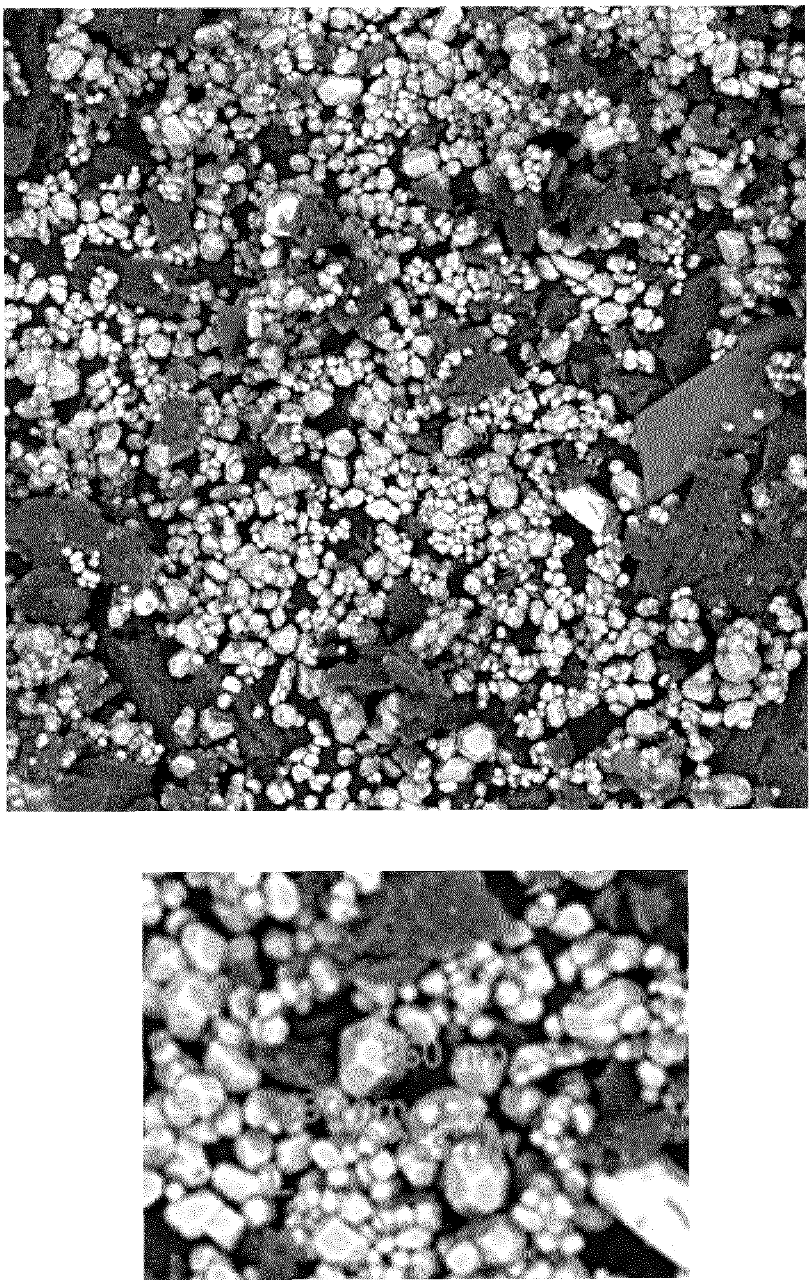

A

B

FIG. 8

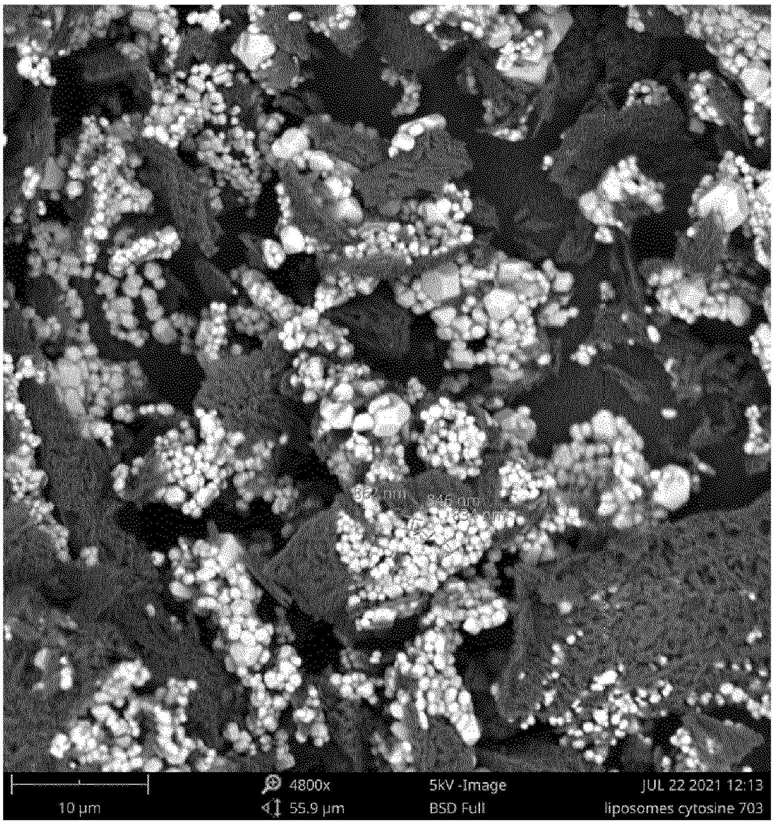
10 µm
4800x
55.9 µm
5kV -Image
BSD Full
JUL 22 2021 12:13
liposomes cytosine 703

A

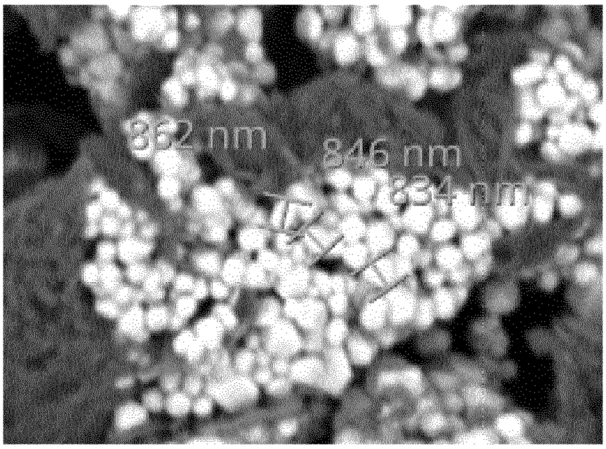
846 nm

B

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017155923 A1
- WO 2015038596 A1
- WO 2010014895 A2
- WO 2006029081 A2
- WO 2011062503 A1
- US 20030026831 A1